# EUROPEAN PATENT APPLICATION

(11) **EP 1 060 732 A2**
(43) Date of publication of application: **20.12.2000**
(21) Application number: 00304542.4
(22) Date of filing: 26.05.2000
(51) Int. Cl.: A61K 7/00, A61K 7/06, A61K 7/48, A61K 9/127

(54) **Novel topical formulations comprising vesicle delivery systems**

(30) Priority: 27.05.1999 US 320894
(71) Applicant: JOHNSON & JOHNSON CONSUMER PRODUCTS, INC., Skillman, New Jersey 08558-9418 (US)
(72) Inventor: Niemiec, Susan M., Yardley, PA 19067 (US); Nystrand, Glenn A., Lebanon, NJ 08833 (US); Wang, Jonas C.T., West Windsor, NJ 08550 (US); Ho, Kie L., Princeton, NJ 08540 (US)
(74) Representative: Mercer, Christopher Paul

(57) **Abstract**

This invention relates to a method for enhancing the transmembrane and/or topical penetration of pharmacologically active substances using a certain vesicle delivery system as an enhancing agent, and an optional detergent, as well as the compositions used therein. Various active agents, such as hair growth agents, hair inhibitor agents, anti-acne agents, depilatory agents, anti-aging agents, and depigmentation agents, may be effectively delivered into the skin, hair follicles and sebaceous glands using the compositions of the present invention.

## Description

### Field of the Invention

The invention relates to novel vesicle compositions containing benefit agents that are capable of effectively depositing the benefit agents into and onto the skin and hair, and methods for improving the deposition of such benefit agents into and onto the skin. The invention further relates to novel cleansing compositions, such as those for the skin and hair and in particular shampoos, comprised of intact, stable vesicles for use in personal cleaning applications.

### Background of the Invention and Prior Art

Therapeutic shampoos comprised of various detergent and therapeutic agents are known in the art. See, e.g. U.S. Pat. No. 5,730,965; U.S. Pat. No. 5,723,112; and U.S. Pat. No. 5,624,666. These shampoos are typically comprised of an anionic surfactant in combination with a therapeutic agent such an chloroxylenol; an antifungal such as ketoconazole; and/or an antidandruff agent such as zinc pyrithione.

In order for antidandruff agents to be most effective, they must not only be capable of relieving the flaking and itching symptoms associated with dandruff, but they also must be substantive to the skin and hair in order to extend the efficacy of the antidandruff agent from one shampoo treatment to the next. Unfortunately, many therapeutic shampoos do not provide sufficient deposition of agents onto the hair fibers and scalp during the shampooing process. Without such deposition, large proportions of therapeutic agents are rinsed away and therefore provide little to no therapeutic benefit.

One way known in the art for improving the deposition of therapeutic agents on the hair or skin is via significantly increasing the levels of therapeutic agents in the shampoo compositions. However, not only does the use of such high levels therapeutic agents disadvantageously increase raw materials costs, but it also reduces the latherability of the shampoo and deleteriously affects product stability.

The presence of detergents in the anti-dandruff shampoos also interferes with the ability of therapeutic agents to deposit onto the hair because they are designed to carry or remove oil, grease, dirt, and particulate matter from the hair and scalp during rinsing. In addition, these detergents, such as anionic surfactants, also adversely affect the hair with an undesirable harsh and dry to the touch feel. Thus, the condition of the hair usually requires a post-shampoo treatment with a conditioning composition to improve the physical characteristics of the hair.

Because many antidandruff particle agents are insoluble in aqueous media, it is difficult to formulate these agents in a stable, aqueous, anionic surfactant-based antidandruff shampoo without the use of inorganic mineral or synthetic or natural polymer or gum suspending agents. Unfortunately, the combination of the antidandruff agents and the suspending agents often adversely affects the foaming characteristics of the shampoo composition. Therefore, there is a need to develop antidandruff compositions that may be stabilized in the absence of such suspending agents.

It is known in the art that the delivery of many benefit agents may be accomplished via the follicular pathway. See, e.g., Scheuplein, *J Invest Dermatol* 48:79-88 (1967); Behl et al.,*J Pharm Sci* 70:835-837 (1981); and Illel et al., *J Pharm Sci* 80:424-427 (1991). Liposomally aided follicular delivery has been investigated as a potential means of directing active agents that affect the hair and pilosebaceous appendages to their sites of action. See, e.g., Dowton et al., *STP Pharma Sciences* 3: 404-407 (1993)(liposomes direct small and large molecules alike into follicles which, once there, can serve as a reservoir for protracted delivery into the dermis); and Niemiec et al. *STP Pharma Sciences* 4: 145-149 (1994).

One object of the present invention is to deliver various active agents into either the: 1) perifollicular space or ducts connected to the perifollicular cell, such as pilosebaceous ducts; and/or 2) the cells from any of the layers of the epidermis and the dermis which are close to a hair shaft; and/or 3) to the hair fibers. Another object of the present invention is to create formulations that contain a relatively low amount of benefit agent but which more effectively deposit benefit agents, such as anti-dandruff agents and conditioners, to the skin, hair, and pilosebaceous units, i.e. hair follicles, sebaceous glands and ducts, without sacrificing latherability or stability of the end product.

### Summary of the Invention

In accordance with the present invention, we have found a composition for enhancing the topical application of benefit agents comprising, consisting essentially of, and/or consisting of, based upon the total weight of the composition,:
A. a vesicle delivery system comprised of
   i. a vesicle bilayer comprised of
      a. at least one first dual chain lipid;
      b. optionally, at least one first single chain lipid;
      c. at least one sterol; and
   ii. a hydrophilic carrier contained within the vesicle bilayer; and
B. an effective amount of the benefit agent.

Another embodiment of this invention is directed to a cleansing composition for enhancing the topical application of benefit agents comprising, consisting essentially of, and/or consisting of, based upon the total weight of the composition,:
A. a vesicle delivery system comprised of
   i. a vesicle bilayer comprised of
      a. at least one first dual chain lipid;
      b. optionally, at least one first single chain lipid;
      c. at least one sterol; and
   ii. a hydrophilic carrier contained within the vesicle bilayer;
B. an effective amount of the benefit agent; and
C. a detergent.

Another embodiment of this invention is directed to a cleansing composition comprising, consisting essentially of, and/or consisting of, based upon the total weight of the composition:
A. a vesicle delivery system comprised of
   i. a vesicle bilayer comprised of
      a. at least one first dual chain lipid;
      b. optionally, at least one first single chain lipid;
      c. at least one sterol; and
   ii. a hydrophilic carrier contained within the vesicle bilayer; and
B. a detergent.

Yet another embodiment of this invention is directed to a method for enhancing the topical application of benefit agents which comprises, consists essentially of, and/or consists of topically administering to a human or animal a composition comprised of:
A. a vesicle delivery system comprised of
   i. a vesicle bilayer comprised of
      a. at least one first dual chain lipid;
      b. optionally, at least one first single chain lipid;
      c. at least one sterol; and
   ii. a hydrophilic carrier contained within the vesicle bilayer; and
B. an effective amount of the benefit agent.

Yet another embodiment of the present invention is directed to a method for treating hair loss comprising, consisting essentially of, and/or consisting of topically administering to a human or animal at a desired area for treating hair loss a composition comprised of, consisting essentially of, and consisting of, based upon the total weight of the composition,:
A. a vesicle delivery system comprised of
   i. a vesicle bilayer comprised of
      a. at least one first dual chain lipid;
      b. optionally, at least one first single chain lipid;
      c. at least one sterol; and
   ii. a hydrophilic carrier contained within the vesicle bilayer; and
B. an effective amount of a hair loss treatment agent.

Yet another embodiment of the present invention is directed to a method for inhibiting hair growth comprising, consisting of, and/or consisting essentially of topically administering to a human or animal at a desired area for hair loss a composition comprised of, consisting of, and consisting essentially of, based upon the total weight of the composition,:
A. a vesicle delivery system comprised of
   i. a vesicle bilayer comprised of
      a. at least one first dual chain lipid;
      b. optionally, at least one first single chain lipid;
      c. at least one sterol; and
   ii. a hydrophilic carrier contained within the vesicle bilayer; and
B. an effective amount of a hair growth inhibiting agent.

Yet another embodiment of the present invention is directed to a method for treating or minimizing the effects of aging comprising, consisting of, and/or consisting essentially of, topically administering to a human or animal at a desired area a composition comprised of, consisting of, and consisting essentially of, based upon the total weight of the composition,:
A. a vesicle delivery system comprised of
   i. a vesicle bilayer comprised of
      a. at least one first dual chain lipid;
      b. optionally, at least one first single chain lipid;
      c. at least one sterol; and
   ii. a hydrophilic carrier contained within the vesicle bilayer; and
B. an effective amount of an anti-aging active agent.

Yet another embodiment of the present invention is directed to a method for treating acne comprising, consisting of, and/or consisting essentially of, topically administering to a human or animal at a desired area a composition comprised of, consisting of, and consisting essentially of, based upon the total weight of the composition,:
A. a vesicle delivery system comprised of
   i. a vesicle bilayer comprised of
      a. at least one first dual chain lipid;
      b. optionally, at least one first single chain lipid;
      c. at least one sterol; and
   ii. a hydrophilic carrier contained within the vesicle bilayer; and
B. an effective amount of an anti-acne active agent.

Yet another embodiment of the present invention is directed to a method for depigmenting skin comprising, consisting of, and/or consisting essentially of, topically administering to a human or animal at a desired area a composition comprised of, consisting of, and consisting essentially of, based upon the total weight of the composition,:
A. a vesicle delivery system comprised of
   i. a vesicle bilayer comprised of
      a. at least one first dual chain lipid;
      b. optionally, at least one first single chain lipid;
      c. at least one sterol; and
   ii. a hydrophilic carrier contained within the vesicle bilayer; and
B. an effective amount of a depigmentation active agent.

Yet another embodiment of the present invention is directed to a method for treating the diseases of dandruff, seborrheic dermatitis, and psoriasis and/or the symptoms associated therewith comprising, consisting of, and/or consisting essentially of topically administering to a human or animal at a desired area a composition comprised of, based upon the total weight of the composition,:
A. a vesicle delivery system comprised of
   i. a vesicle bilayer comprised of
      a. at least one first dual chain lipid;
      b. optionally, at least one first single chain lipid;
      c. at least one sterol; and
   ii. a hydrophilic carrier contained within the vesicle bilayer; and
B. an effective amount of a benefit agent selected from the group consisting of an anti-dandruff agent, an anti-seborrheic dermatitis agent, an anti-psoriasis agent, and mixtures thereof.

The combination of the benefit agents with the nonionic vesicles or cationic/nonionic vesicles results in formulations that are uniquely effective in depositing the benefit agents into and onto the skin, hair and pilosebaceous units. Similarly, when the above formulation is further combined with detergents, the resulting cleansing formulation not only effectively deposits the benefits agents into and onto the skin, hair, and pilosebaceous units after rinsing, but also remains stable.

### Brief Description of the Drawings

The invention will be more fully understood and further advantages will become apparent when reference is made to the following detailed description of the invention and the accompanying drawing in which:
Figure 1 is a transmission electron photomicrograph at a magnification of 86,000X of nonionic liposome composition upon accelerating aging at 50°C for 4 weeks.
Figure 2 is a transmission electron photomicrograph at a magnification of 108,000X of nonionic liposomal shampoo composition upon accelerating aging at 50°C for 4 weeks.
Figure 3 is a transmission electron photomicrograph at a magnification of 80,000X of low cationic/nonionic liposomal shampoo composition upon accelerating aging at 50°C for 4 weeks.
Figure 4 is a transmission electron photomicrograph at a magnification of 27,000X of high cationic/nonionic liposomal shampoo composition upon accelerating aging at 50°C for 4 weeks.
Figure 5 is a scanning electron photomicrograph of hair fibers at the middle and ends of hair tresses washed 10 times with an anionic-base shampoo at an at a magnification of 2000 and 5000X.
Figure 6 is a scanning electron photomicrograph of hair fibers obtained from the middle and ends of hair tresses after the tresses were washed 10 times with a high cationic/nonionic liposomal-shampoo (anionic base) composition at a at a magnification of 2000 and 5000X.
Figure 7 is a scanning electron photomicrograph of hair fibers at the middle and ends of hair tresses washed 10 times with a commercially-known, zinc pyrithione-containing anti-dandruff shampoo at a magnification 2000 and 5000X.

### Detailed Description of the Invention

As used herein, the term, "vesicle" includes liposomes and other multi-lamellar structures. By "first" dual chain lipid, it is meant a dual chain lipid having either a cationic or nonionic charge. Similarly, by "first" single chain lipid, it is meant a single chain lipid having either a cationic or nonionic charge. By "second" dual chain lipid, it is meant a dual chain lipid having either: 1) a cationic charge if the first dual chain lipid possesses a nonionic charge, or 2) a nonionic charge if the first dual chain lipid possesses a cationic charge. By "second" single chain lipid, it is meant a single chain lipid having either: 1) a cationic charge if the first single chain lipid possesses a nonionic charge, or 2) a nonionic charge if the first single chain lipid possesses a cationic charge. "Vesicle delivery system," as used herein, shall include all of the lipid and sterol components that comprise the bilayers as well as the internal, encapsulated hydrophilic component.

The compositions of the present invention incorporate a vesicle delivery system for topically delivering benefit agents into and/or onto the skin and hair, in the optional presence of detergents, whereby the vesicle system is comprised of: 1) at least one first dual chain lipid; 2) at least one first single chain lipid; 3) a sterol; 4) a hydrophilic carrier; and 5) optionally, at least one second dual chain lipid and/or at least one second single chain lipid. The vesicles of the present invention are comprised of an enclosed, exterior bilayer structure comprised of components 1 through 4 and 5, with the hydrophilic carrier being contained therein. The vesicle delivery system may be classified as having an overall charge that is either nonionic, cationic, or cationic/nonionic, depending upon the selection of lipids of which it is comprised.

The first component of the vesicle delivery system is a first dual chain lipid, which may be comprised of one or more nonionic dual chain lipids, one or more cationic dual chain lipids, or mixtures thereof. As used herein, the terms "dual chain lipid" are used interchangeably herein with the terms "primary wall material" and the terms "primary lipid." Dual chain lipids are comprised of a polar head group and two hydrophobic chains. The dual chain lipids constitute the greatest proportion (by weight) of the bilayer-forming components of the vesicle, and preferably are present in an amount, based upon the total weight of such bilayer-forming components, of about 40% or greater, and preferably from about 40% to about 95%.

Examples of suitable nonionic dual chain lipids include nonexclusively, glyceryl diesters, alkoxylated amides, and mixtures thereof.

Examples of suitable glyceryl diesters preferably include those glyceryl diesters having from about 10 carbon atoms to about 30 carbon atoms, and more preferably from about 12 carbon atoms to about 20 carbon atoms. Preferred glyceryl diesters include, but are not limited to glyceryl dilaurate ("GDL"), glyceryl dioleate, glyceryl dimyristate, glyceryl distearate ("GDS"), glyceryl sesuioleate, glyceryl stearate lactate, and mixtures thereof, with glyceryl dilaurate, glyceryl distearate and glyceryl dimyristate being more preferred.

Examples of suitable alkoxylated amides include those which conform to the structure shown below in Formula I.: 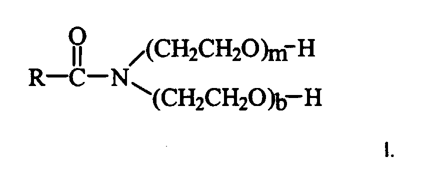 wherein R is a unbranched alkyl group having from about 8 carbon atoms to about 30 carbon atoms, and preferably from about 12 carbon atoms to about 24 carbon atoms, m is an integer of from about 0 to about 100, and b is an integer of from about 0 to about 100, with the proviso that the sum of m plus b is from about 8 to about 100. An exemplary member of this class is known by the CTFA name "PEG-6 Cocoamide," which conforms generally to structure I. above wherein RCO represents the fatty acids derived from coconut oil and both m and b, respectively, have an average value of about 6.

Examples of suitable cationic dual chain lipids include nonexclusively those bilayer-forming cationic lipids that contain two unsaturated fatty acid chains having from about 10 to about 26 carbon atoms such as di(soyoylethyl) hydroxyethylmonium methosulfate (DSHM); N-[I-(2,3-dioleyloxy)propyl]-N,N,N-trimethylammonium bromide (DOTMA); 1,2-dimyristytoxypropyl-N,N-dimethyl-hydroxyethyl ammonium bromide (DMRIE); [N-(N, N' - dimethylaminoethane) carbamoyl] cholesterol (DC-Chol); dioctadecylamidoglycyl spermidine (DOGS); dimethyl dioctadecylammonium bromide (DDAB); dioleoyl phosphatidylethanolamine (DOPE); 2,3-dioleoyloxyl-N[2(sperminecarbozamide-O-ethyl]-N,N-dimethyl-propanaminium trifluoroacetate (DOSPA); I-[2-(oleoyloxy)-ethyl]-2-oleyl-3-(2hydroxyethyl) imidazolinium chloride (DOTIM); 1,2-dioleoyloxy-3-(trimethylammonio) propane (DOTAP); 1,2-diacyl-3-trimethylammonium propane (TAP); 1,2-diacyl-3-dimethylammonium propane (DAP); fatty acid salts of quaternary amines such as dicocodimonium chloride (Quaternium 34), quaternary dimethyldiacyl amines wherein the acyl groups have from about 8 carbon atoms to about 30 carbon atoms, and preferably from about 10 carbon atoms to about 24 carbonatoms, and derivatives and mixtures thereof such as ammonium derivatives, i.e. dimethyl dihydrogenated tallow ammonium chloride (Quaternium 18), decyl dimethyl octyl ammonium chloride (Quaternium 24) and mixtures thereof, with di(soyoylethyl) hydroxyethylmonium methosulfate (DSHM) being more preferred. Other suitable cationic dual chain lipids are further described in the following references: Fasbender et al., 269 *Am J Physiol* L45-L5 1 (1995); Solodin et al., 34 *Biochemistry* 13537-13544 (1995); Felgner et at., 269 *J Biol Chem* 2550-2561(1994); Stamatatos et al., 27 *Biochemistry* 3917 - 3925 (1988); and Leventis and Silvius, 1023 *Biochim Biophys Acta* 124-132 (1990), which are all incorporated by reference herein.

Several of these cationic dual chain lipids, such as TAP and DAP, may possess a variety of types of chain groups having carbon atom:number of saturated bonds ratios of, for example, 14:0; 16:0; 18:0; and 18:1 as well as a variety of types of acyl groups having from about 10 carbon atoms to about 18 carbon atoms such as dimyristoyl; dipalmitoyl; distearoyl; and dioleoyl.

Other dual chain lipids are also considered to be within the scope of the invention so long as they form either a nonionic lipid vesicle, a cationic lipid vesicle, or a nonionic/cationic lipid vesicle with the single chain lipid and sterol, either alone in the presence of other reagents such as, e.g. antioxidants, preservatives, buffering agents, chelating agents, UV stabilizers, and mixtures thereof.

The amount of nonionic dual chain lipids in the vesicle bilayer may range from, based upon the total weight of the dual chain lipids in the vesicle bilayer, from about 0 percent to about 95 percent, and preferably from about 10 percent to about 65 percent. The amount of cationic dual chain lipids in the vesicle bilayer may range from, based upon the total weight of the dual chain lipids in the vesicle bilayer, from about 0 percent to about 95 percent, and preferably from about 1 percent to about 50 percent. In embodiments wherein the vesicle bilayer is comprised of both cationic dual chain lipids and nonionic dual chain lipids, the amount of cationic dual chain lipids and nonionic dual chain lipids present is, based upon the total weight of cationic dual chain lipids and nonionic dual chain lipids, from about 1 percent to about 50 percent, and preferably from about 2 percent to about 30 percent of cationic dual chain lipid, and from about 50 percent to about 99 percent, and preferably from about 70 percent to about 98 percent of nonionic dual chain lipid.

The second component in the vesicle system is a single chain lipid, which may be comprised of a non-ionic single chain lipid, a cationic single chain lipid, or a nonionic/cationic single chain lipid. The single chain lipids are present in the vesicle in an amount, based upon the total weight of the vesicle bilayer, from about 1 percent to about 50 percent, and preferably from about 2 percent to about 30 percent.

Suitable nonionic single chain lipids are comprised of a polar head group and one fatty acid chain and are also known as either "secondary wall materials" or "secondary lipids." Examples of suitable nonionic single chain lipids include, but are not limited to glyceryl monoesters; polyoxyethylene fatty ethers wherein the polyoxyethylene head group has from about 2 to about 100 groups and the fatty acid tail group has from about 10 to about 26 carbon atoms; alkoxylated alcohols wherein the alkoxy group has from about 1 carbon atoms to about 200 carbon atoms and the fatty alkyl group has from about 8 carbon atom to about 30 carbon atoms, and preferably from about 10 carbon atoms to about 24 carbon atoms; alkoxylated alkyl phenols wherein the alkoxy group has from about 1 carbon atoms to about 200 carbon atoms and the fatty alkyl group has from about 8 carbon atom to about 30 carbon atoms, and preferably from about 10 carbon atoms to about 24 carbon atoms; polyoxyethylene derivatives of polyol esters; alkoxylated acids wherein the alkoxy group has from about 1 carbon atoms to about 200 carbon atoms and the fatty alkyl group has from about 8 carbon atom to about 30 carbon atoms, and preferably from about 10 carbon atoms to about 24 carbon atoms; and mixtures thereof.

Examples of suitable glyceryl monoester nonionic single chain lipids preferably include those glyceryl monoesters having from about 10 carbon atoms to about 30 carbon atoms, and more preferably from about 12 carbon atoms to about 20 carbon atoms, and mixtures thereof. More preferred glyceryl monoesters include glyceryl caprate, glyceryl caprylate, glyceryl cocoate, glyceryl erucate, glyceryl hydroxystearate, glyceryl isostearate, glyceryl lanolate, glyceryl laurate, glyceryl linolate, glyceryl myristate, glyceryl oleate, glyceryl PABA, glyceryl palmitate, glyceryl ricinoleate, glyceryl stearate, glyceryl thiglycolate, and mixtures thereof, with glyceryl laurate and glyceryl myristate being most preferred.

Examples of suitable polyoxyethylene fatty ether nonionic single chain lipids include polyoxyethylene cetyl ether, polyoxyethylene stearyl ether, polyoxyethylene cholesterol ether, polyoxyethylene laurate, polyoxyethylene dilaurate, polyoxyethylene stearate, polyoxyethylene distearate, polyoxyethylene lauryl ether, polyoxyethylene stearyl ether, and mixtures thereof. Preferred polyoxyethylene fatty ethers include polyoxyethylene stearyl ether, polyoxyethylene myristyl ether, polyoxyethylene lauryl ether, and mixtures thereof, with each ether having from about 3 to about 10 oxyethylene units.

Suitable examples of an alkoxylated alcohol nonionic single chain lipid includes those that are useful as nonionic surfactants and have the structure shown in formula II below:

R₅―(OCH₂CH₂)y―OH II.

wherein R₅ is an unbranched alkyl group having from about 10 to about 24 carbon atoms and y is an integer between about 4 and about 100, and preferably between about 10 and about 100. A preferred alkoxylated alcohol is the species wherein R₅ is a lauryl group and y has an average value of 23, which is known by the CTFA name "laureth 23" and is available from Uniqema, Inc. of Wilmington, Delaware under the tradename, "BRIJ 35."

Suitable examples of an alkoxylated alkyl phenols nonionic single chain lipid includes those which generally conform to the structure shown in formula III below: 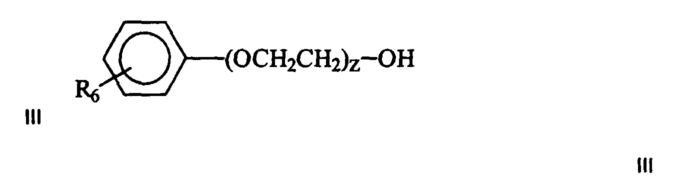 wherein R₆ is an unbranched alkyl group having from about 10 to about 24 carbon atoms and z is an integer of from about 7 and 120, and preferably from about 10 and about 100. An especially preferred member of this class of materials is the species wherein R₆ is a nonyl group and z has an average value of about 14. This material is known by the CTFA name "nonoxynol-14" and is available under the tradename, "MAKON 14" from the Stepan Company of Northfield, Illinois.

Suitable polyoxyethylene derivatives of polyol ester single chain nonionic lipids are those wherein the polyoxyethylene derivative of polyol ester (1) is derived from (a) a fatty acid containing from about 8 to about 22, and preferably from about 10 to about 14 carbon atoms, and (b) a polyol selected from sorbitol, sorbitan, glucose, α-methyl glucoside, polyglucose having an average of about 1 to about 3 glucose residues per molecule, glycerine, pentaerythritol and mixtures thereof, (2) contains an average of from about 10 to about 120, and preferably about 20 to about 80 oxyethylene units; and (3) has an average of about 1 to about 3 fatty acid residues per mole of polyoxyethylene derivative of polyol ester.

Examples of preferred polyoxyethylene derivatives of polyol esters include, but are not limited to PEG-80 sorbitan laurate and Polysorbate 20. PEG-80 sorbitan laurate, which is a sorbitan monoester of lauric acid ethoxylated with an average of about 80 moles of ethylene oxide, is available commercially from ICI Surfactants of Wilmington, Delaware under the tradename, "Atlas G-4280." Polysorbate 20, which is the laurate monoester of a mixture of sorbitol and sorbitol anhydrides condensed with approximately 20 moles of ethylene oxide, is available commercially from ICI Surfactants of Wilmington, Delaware under the tradename "Tween 20." Another exemplary polyol ester is sorbitan stearate, which is available from Uniqema, Inc. under the tradename, "SPAN 60."

Suitable examples of alkoxylated acid single chain, nonionic lipids include the esters of an acid, most usually a fatty acid, with a polyalkylene glycol. An exemplary material of this class has the CTFA name "PEG-8 laurate," and the following structure shown in formula IV:

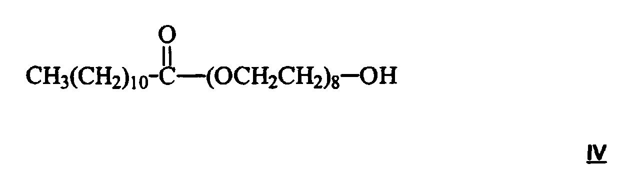

Most preferred single chain nonionic lipids include polyoxyethylene fatty ethers, glyceryl monoesters, and mixtures thereof, with polyoxyethylene stearyl ether, polyoxyethylene myristyl ether, polyoxyethylene lauryl ether, glyceryl laurate, glyceryl myristate, glyceryl stearate, and mixtures thereof, whereby each ether has from about 5 to about 10 oxyethylene units, being more preferred.

Examples of suitable cationic single chain lipids nonexclusively include quaternary trimethylmonoacyl amines, wherein the acyl groups have from about 8 carbon atoms to about 30 carbon atoms, and preferably from about 10 carbon atoms to about 24 carbon atoms, and derivatives and mixtures thereof such as ammonium derivatives, i.e. stearamidopropyl dimethyl (myristyl acetate) ammonium chloride (Quaternium 70), triethyl hydrogenated tallow ammonium chloride (Quaternium 16), benzalkonium chloride and derivatives and mixtures thereof.

Other single chain lipids are also considered to be within the scope of the invention so long as they form either a nonionic lipid vesicle, a cationic lipid vesicle, or a nonionic/cationic lipid vesicle with the dual chain lipid, either alone in the presence of other reagents such as, e.g. antioxidants, preservatives, buffering agents, chelating agents, UV stabilizers, and mixtures thereof.

The amount of nonionic single chain lipids in the vesicle bilayer may range from, based upon the total weight of the single chain lipids in the vesicle bilayer, from about 0 percent to about 70 percent, and preferably from about 1 percent to about 30 percent. The amount of cationic single chain lipids in the vesicle bilayer may range from, based upon the total weight of the single chain lipids in the vesicle bilayer, from about 0 percent to about 70 percent, and preferably from about 1 percent to about 30 percent. In embodiments wherein the vesicle bilayer is comprised of both cationic single chain lipids and nonionic single chain lipids, the amount of cationic single chain lipids and nonionic single chain lipids may range, based upon the total weight of cationic single chain lipids and nonionic single chain lipids, from about 1 percent to about 50 percent, and preferably from about 5 percent to about 20 percent of cationic single chain lipids, and from about 1 percent to about 50 percent, and preferably from about 5 percent to about 20 percent of a nonionic single chain lipid. The choice of an optional second dual chain lipid and/or an optional second single chain lipid for inclusion in the vesicle delivery system is dependent upon the optional lipid's respective ability to complex with the hair and skin surface and its compatibility with the lipid vesicle itself. In general, when using any of the above lipids, it is preferred to use only such lipids that do not degrade the nonionic vesicle delivery system and that are capable of complexing to form an entity having negative charges when combined with a nonionic lipid vesicle.

The third component of the vesicle is a charge producing agent that includes but is not limited to sterols. Examples of suitable sterols include cholesterol and salts and esters thereof; phytocholesterol, hydrocortisone, alpha-tocopherol, betasitosterol, bisabolol and mixtures thereof, with cholesterol being most preferred.

The fourth component of the vesicle is a hydrophilic component such as water, polar solvents, or mixtures thereof. Examples of polar solvents nonexclusively include glycols such as glycerin, alcohols and preferably those having from about 2 carbon atoms to about 6 carbon atoms, propylene glycol, sorbitol, oxyalkylene polymers such as PEG 4, and mixtures thereof.

Various combinations and ratios of first single chain lipids, first dual chain lipids, optional second single chain lipids, optional dual chain lipids, sterols, hydrophilic components, and other optional additional agents may be used for preparing a vesicle delivery system of the invention. While the components of the vesicle delivery system and their respective weight ratio content therein may depend upon, for example, the final characteristics desired in the vesicle system, the properties of the different components in the system, the desired use(s) of the vesicle delivery system, and/or the type of non-vesicle components to be used with the vesicle delivery system, the preferred amounts of components are, based upon the total vesicle delivery system, from about 40 percent to about 95 percent, and more preferably from about 40 percent to about 60 percent of first dual chain lipids; from about 0 percent to about 50 percent, and more preferably from about 1 percent to about 20 percent optional second dual chain lipids; from about 1 percent to about 55 percent, and more preferably from about 1 percent to about 35 percent first single chain lipids; from about 0 percent to about 50 percent, and more preferably from about 1 percent to about 20 percent optional second single chain lipids; from about 1 percent to about 50 percent, and more preferably from about 1 percent to about 25 percent sterol; and from about 50 percent to about 99 percent, and more preferably from about 60 percent to about 90 percent hydrophilic component.

A particularly preferred non-ionic vesicle of the invention contains, based upon the total weight of the vesicle bilayers, from about 40 percent to about 60 percent, and preferably from about 40 percent to about 50 percent of a glyceryl distearate dual chain lipid; from about 10 percent to about 45 percent, and preferably from about 10 percent to about 20 percent of a polyoxyethylene-10-stearyl ether single chain lipid, and from about 5 percent to about 45 percent, and preferably from about 5 percent to about 25 percent of cholesterol. Similar non-ionic vesicles are described in United States Patent No. 4,911,928, United States Patent No. 5,032,457, United States Patent No. 5,147,723, and United States Patent No. 5,260,065, the disclosures which are all incorporated herein by reference in their totalities.

In another particularly preferred embodiment, the nonionic vesicles are comprised of, based upon the total weight of the vesicle bilayers, from about 45 to about 55 percent of glyceryl distearate, from about 1 percent to about 50 percent, and preferably 'from about 5 percent to about 25 percent of cholesterol, and from about 18 percent to about 28 percent of polyoxyethylene-10-stearyl ether.

A preferred nonionic/cationic vesicle is comprised of, based upon the total weight of the vesicle bilayer, from about 25 percent to about 95 percent, and preferably from about 30 percent to about 65 percent glyceryl distearate dual chain lipid; from about 1 percent to about 45 percent, and preferably from about 5 percent to about 35 percent of a polyoxyethylene-10-stearyl ether single chain lipid, from about 1 percent to about 40 percent, and preferably from about 5 percent to about 25 percent of a cholesterol sterol, and from about 1 percent to about 45 percent, and preferably from about 2 percent to about 25 percent of a di(soyoylethyl) hydroxyethylmonium methosulfate (DSHM) cationic dual chain lipid.

In another preferred embodiment, the nonionic/cationic vesicle is comprised of, based upon the total weight of the vesicle bilayers, from about 25 percent to about 60 percent, and more preferably from about 23 percent to about 27 percent of a nonionic dual chain lipid such as glyceryl dilaurate ("GDL"); from about 5 percent to about 45 percent, and more preferably from about 23 percent to about 27 percent of another nonionic dual chain lipid such as glyceryl distearate ("GDS"); from about 1 percent to about 40 percent, and more preferably from about 13 percent to about 17 percent of a sterol such as cholesterol, from about 5 percent to about 40 percent, and more preferably from about 20 percent to about 25 percent of a nonionic single chain lipid such as polyoxyethylene-10-stearyl ether, and from about 1 percent to about 45 percent, and more preferably from about 10 percent to about 15 percent of a cationic dual chain lipid such as di(soyoylethyl) hydroxyethylmonium methosulfate. Without being bound by theory, when the ratio of GDL:GDS is varied such that the amount of GDL exceeds that of GDS, the "penetration enhancer" effect is dominated by the excess GDL-based formulation. As used herein, "penetration enhancer effect" is the transport of active agents across the stratum corneum into the living skin tissues. Conversely, when the amount of GDS exceeds that of GDL, the "reservoir effect" is dominated by the excess GDS-based formulation. As used herein, "reservoir effect" is the accumulation or retention of active agents in the stratum corneum over time. It is preferable to use certain benefit agents, such as antibacterial agents, insecticides, humectants, and sunscreens, as well as surfactants in vesicles possessing reservoir effect properties. Conversely, it is more preferable to use other benefit agents, such as depigmentation agents, anti-acne agents, anti-dandruff agents, anti-psoriasis agents, anti hair loss agents in vesicles possessing penetration effect properties.

Nonionic vesicles may preferably be prepared by mixing appropriate amounts of the single chain lipids, the dual chain lipids, and sterols under conditions sufficient to produce a homogeneous mixture. While the temperature for mixing may depend upon, for example, the melting points of the predominate lipids, typically the nonionic vesicle systems may be prepared under temperatures of from about 65 °C to about 80 °C and under ambient pressure conditions. In order to produce a vesicle system having improved consistency, it is more preferable to mix the single chain lipids, the dual chain lipids, and the sterols under high shear in, for example, an apparatus as described in United States Patent No. 5,013,497, which is incorporated by reference herein.

Nonionic/cationic vesicles may be prepared by first preparing the nonionic lipid vesicle as described above, followed by mixing the cationic lipids therewith at room temperature or at the phase transition temperature of the nonionic lipids in a mixer, such as a Caframo mixer. In a preferred alternative embodiment, the dual chain lipids, the single chain lipids, the sterols and the cationic lipids may be mixed simultaneously *using the* apparatus as described in United States Patent No. 5,013,497.

Additional optional agents, such as, for example, other lipids, benefit agents, and/ or chemical reagents such as buffers, may be added to the vesicles at any time during its preparation. Generally, these optional agents may reside in either: 1) the interior hydrophilic component of the vesicle; 2) in the vesicle bilayers; or 3) external to the vesicle. When using hydrophobic benefit agents such as elubiol, ketoconazole, retinol, benzoyl peroxide, retinoic acid, and the like, it is preferable to combine such benefit agents with the vesicle components to ensure that the benefit agents are in the vesicle bilayers. When using hydrophilic benefit agents such as alpha hydroxy acids, beta hydroxy acids such as salicylic acid, zinc pyrithione (as a particulate), ascorbic acid, it is also preferable to combine such benefit agents with the vesicle components to ensure that the benefit agents will result in the hydrophilic component of the vesicle.

In an alternative embodiment where it is desired to have the benefit agents external to the vesicle, e.g. present in an exterior surfactant mixture as opposed to in the actual vesicles themselves, it is preferable to first combine the benefit agent with the detergent mixture, then combine the resulting mixture with the other vesicle components. This method may preferable for benefit agents such zinc pyrithione. We have unexpectedly found that such vesicle systems having the benefit agents present in the surfactant mixture displayed enhanced delivery of the benefit agent into the pilosebaceous units even through the benefit agent was not entrapped in the vesicles. Hence, enhanced deposition of the benefit agent may be achieved regardless of whether the benefit agent is incorporated within the vesicles, in the phase external to the vesicles, or within the vesicle bilayers.

Another embodiment of this invention is directed to a composition comprising the above-described vesicle delivery system and a benefit agent, whereby the vesicle delivery system is capable of effectively enhancing the topical delivery of the benefit agents into and onto the skin and/or the pilosebaceous units. By "benefit agent," it is mean any active ingredient that is to be delivered into and/or onto the skin at a desired location, such as a cosmetic agent or a pharmaceutical agent. By "cosmetic agent," it is meant any ingredient that is appropriate for cosmetically treating, providing nutrients to, and/or conditioning the hair and/or skin via topical application. By pharmaceutical agent," it is mean any drug that is either hydrophobic or hydrophilic in nature and appropriate for topical use. As used herein "medicament agents" include those agents capable of promoting recovery from injury and illness.

Examples of suitable benefit agents include, but are not limited to, antimicrobial agents; allergy inhibitors; anti-acne agents; anti-aging agents; antiseptics; analgesics; antitussives; antipruritics; local anesthetics; anti-hair loss agents; antihistamines; aritiinfectives; inflammation inhibitors; anti-emetics; anticholinergics; vasoconstrictors; vasodilators; wound healing promoters; peptides, polypeptides and proteins; deodorants and anti-perspirants; medicament agents; skin emollients and skin moisturizers; hair conditioners; hair softeners; hair moisturizers; vitamins; tanning agents; skin lightening agents; antifungals such as antifungals for foot preparations; hair growth inhibitors, anti-dandruff agents, anti-seborrheic dermatitis agents, anti-psoriasis agents, hair loss promoters, depigmentation agents, depilating agents; shaving preparations; external analgesics; perfumes; counterirritants; hemorrhoidals; insecticides; poison ivy products; poison oak products; bum products; anti- diaper rash agents; prickly heat agents; make-up preparations; vitamins; amino acids and their derivatives; herbal extracts; retinoids; flavoids; sensates: anti-oxidants; skin conditioners; hair lighteners; chelating agents; cell turnover enhancers; coloring agents; pigments: sunscreens and the like, and mixtures thereof.

Examples of suitable vitamins nonexclusively include vitamin B complex; including thiamine, nicotinic acid, biotin, pantothenic acid, choline, riboflavin, vitamin B6, vitamin B12, pyridoxine, inositol, carnitine; vitamins A,C,D,E,K and their derivatives such as pro-vitamins, and mixtures thereof.

Examples of suitable antibacterial agents nonexclusively include bacitracin, erythromycin, neomycin, tetracycline, chlortetracycline, benzethonium chloride, phenol, and mixtures thereof.

Examples of suitable skin emollients and skin moisturizers nonexclusively include mineral oil, lanolin, vegetable oils, isostearyl isostearate, glyceryl laurate, methyl gluceth 10, methyl gluceth 20 chitosan, and mixtures thereof.

Examples of suitable hair conditioners nonexclusively include quaternized compounds such as behenamidopropyl PG-dimonium chloride, tricetylammonium chloride, dihydrogenated tallowamidoethyl hydroxyethylmonium methosulfate, and mixtures thereof as well as lipophilic compounds like cetyl alcohol, stearyl alcohol, hydrogenated polydecene, and mixtures thereof.

An example of a suitable hair softener nonexclusively includes silicone compounds, such as those that are either non-volatile or volatile and those that are water soluble or water insoluble.

Examples of suitable hair moisturizers nonexclusively include panthenol, panthenyl ethyl ether, pythantriol, and mixtures thereof.

Examples of sunscreen agents nonexclusively include butyl methoxydibenzoylmethane, octyl methoxycinnamate, oxybenzone, octocrylene, octyl salicylate, phenylbenzimidazole sulfonic acid, ethyl hydroxypropyl aminobenzoate, menthyl anthranilate, and mixtures thereof.

An example of a suitable tanning agent nonexclusively includes dihydroxyacetone.

Examples of skin lightening agents nonexclusively include hydroquinone, catechol and its derivatives, ascorbic acid and its derivatives, and mixtures thereof.

Examples of suitable insectides (including insect repellents, anti-scabies and anti-lice treatments) nonexclusively include permethrin, *pyrethrin* , piperonyl butoxide, imidacloprid, N,N-diethyl toluamide, which refers to the material containing predominantly the *meta* isomer, i.e., N,N-diethyl-*m*-toluamide, which is also known as DEET; compounds of the formula V 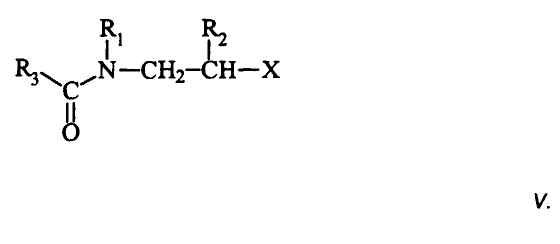 wherein
R₁ is a branched or unbranched alkyl group having about 1 to about 6 carbon atoms;
R₂ is H, methyl or ethyl;
R₃ is a branched or unbranched alkyl or alkoxy group having from about 1 to about 8 carbon atoms; and
X is a -CN or a -COOR₄ group, wherein
   R₄ is a branched or unbranched alkyl group having from about 1 to about 6 carbon atoms,
natural or synthetic pyrethroids, whereby the natural pyrethroids are contained in pyrethrum, the extract of the ground flowers of *Chrysanthemum cinerariaefolium* or *C coccineum*; and mixtures thereof. Within the structure of Formula V. are ethyl 3-(N-butylacetamido)propionate, wherein R₃ is a CH₃ group, R₁ is an n-butyl group, R₂ is H, X is COOR₄ and R₄ is ethyl, which is available commercially from Merck KGaA of Darmstadt, Germany under the name, "Insect Repellent 3535."

An example of an anti fungal for foot preparations nonexclusively includes tolnaftate.

Examples of suitable depilating agents nonexclusively include calcium thioglycolate, magnesium thioglycolate, potassium thioglycolate, strontium thioglycolate, and mixtures thereof.

Examples of suitable external analgesics and local anesthetics nonexclusively include benzocaine, dibucaine, benzyl alcohol, camphor, capsaicin, capsicum, capsicum oleoresin; juniper tar, menthol, menthyl nicotinate, methyl salicylate, phenol, resorcinol, turpentine oil, and mixtures thereof.

Examples of suitable antiperspirants and deodorants nonexclusively include aluminium chlorohydrates, aluminium zirconium chlorohydrates, and mixtures thereof.

Examples of suitable counterirritants nonexclusively include camphor, menthol, methyl salicylate, peppermint and clove oils, ichtammol, and mixtures thereof.

An example of a suitable inflammation inhibitor nonexclusively includes hydrocortisone.

Examples of suitable hemorrhoidal products nonexclusively include the anesthetics such as benzocaine, pramoxine hydrochloride, and mixtures thereof; antiseptics such as benzethonium chloride; astringents such as zinc oxide, bismuth subgallate, balsam Peru, and mixtures thereof; skin protectants such as cod liver oil, vegetable oil, and mixtures thereof.

Examples of suitable make-up preparations nonexclusively include components for lipstick, rouge, blush, eye liner, eyeshadow powder, mascara, face powder, and mixtures thereof.

Preferred benefit agents nonexclusively include elubiol, 6-(1-piperidinyl)-2,4-pyrimidinediamine-3-oxide, finasteride, ketoconazole, salicylic acid, zinc pyrithione, coal tar, benzoyl peroxide, selenium sulfide, hydrocortisone, sulfur, menthol, pramoxine hydrochloride, tricetylammonium chloride, polyquaternium 10, panthenol, panthenol triacetate, vitamin A and derivatives thereof, vitamin B and derivatives thereof, vitamin C and derivatives thereof, vitamin D and derivatives thereof, vitamin E and derivatives thereof, vitamin K and derivatives thereof, keratin, lysine, arginine, hydrolyzed wheat proteins, hydrolyzed silk proteins, octyl methoxycinnamate, oxybenzone, minoxidil, titanium dioxide, zinc dioxide, retinol, erthromycin, tretinoin, and mixtures thereof.

One preferred type of benefit agent includes those therapeutic components that are effective in the treatment of dandruff, seborrheic dermatitis, and psoriasis. Examples of such suitable benefit agents nonexclusively include zinc pyrithione, selenium sulfide, sulfur; salicylic acid; coal tar; povidone-iodine, imidazoles such as ketoconazole, dichlorophenyl imidazolodioxalan, which is commercially available from Janssen Pharmaceutica, N.V., under the tradename, "Elubiol", clotrimazole, itraconazole, miconazole, climbazole, tioconazole, sulconazole, butoconazole, fluconazole, miconazolenitrite and any possible stereo isomers and derivatives thereof such as anthralin; piroctone olamine (Octopirox); selenium sulfide; ciclopirox olamine; anti-psoriasis agents such as vitamin D analogs, e.g. calcipotriol, calcitriol, and tacaleitrol; vitamin A analogs such as esters of vitamin A, e.g. vitamin A palmitate, retinoids, retinols, and retinoic acid; corticosteroids such as hydrocortisone, clobetasone, butyrate, clobetasol propionate and mixtures thereof.

The amount of benefit agent to be combined with the vesicle delivery system may vary depending upon, for example, the resulting benefit desired and the sensitivity of the user to the benefit agent. However, typically, the benefit agent/vesicle delivery system composition contains, based upon the total weight of the composition, from about 0.001 percent to about 20 percent, and preferably from about 0.01 percent to about 5 percent of the benefit agent, and from about 0.06 percent to about 60 percent, and preferably from about 0.6 percent to about 30 percent of the vesicle delivery system.

We have unexpectedly found that the above-described vesicle delivery system, which can either be nonionic or cationic/nonionic in nature, is capable of efficiently mediating the deposition and permeation of various benefit agents, such as antidandruff agents, onto and into the skin following topical administration thereto.

In yet another embodiment of the present invention is directed to a composition comprised of the vesicle delivery system, the benefit agent, and a detergent. By "detergent," it is meant any known surfactant and/or soap that is compatible with the vesicle delivery system and may nonexclusively include anionic surfactants, nonionic surfactants, cationic surfactants, amphoteric surfactants (including betaine surfactants and zwitterionic surfactants) and mixtures thereof.

Examples of suitable anionic surfactants include, but are not limited to, compounds in classes known as alkyl sulfates, sulfate esters of an alkylphenoxy polyoxyethylene ethanol, alpha-olefin sulfonates, betaalkyloxy alkane sulfonates, alkyl arylsulfonates, alkyl carbonates, alkyl ether carboxylates, fatty acids, sulfosuccinates, alkyl ether sulfosuccinates, sarcosinates, octoxynol phosphates, nonoxynol phosphates, taurates, fatty taurides, sulfated monoglycerides, fatty acid amido polyoxyethylene sulfates, and isethionates and mixtures thereof. Many additional surfactants are described in WO 07/26860 and in McCUTCHEON'S DETERGENTS AND EMULSIFIERS (1989), which are both incorporated herein by reference. These anionic surfactants are generally present in the composition as a neutralized salt in the form of sodium salts, potassium salts, ammonium salts, lithium salts, alkyl ammonium salts, or hydroxyalkyl ammonium salts. Preferred anionic surfactants are alkyl sulfates, alkyl ether sulfates, alkyl phosphates, amino acid salts such as N-acyl-L-glutamate, α-olefin sulfonates, alkyl sarcosinates, alkyl benzene sulfonates, acyl isethionates, alkyl sulfosuccinates, acyl methyl taurides, and mixtures thereof, with sodium C14 - 16 olefin sulfonate, ammonium lauryl sulfate, sodium trideceth sulfate, sodium laureth sulfate, disodium laureth sulfosuccinate being most preferred.

Examples of suitable nonionic surfactants include, but are not limited to, those set forth in WO 07/26860, with polysorbate 20, long chain alkyl glucosides having alkyl groups containing about 8 carbon atoms to about 22 carbon atoms; coconut fatty acid monoethanolamides such as cocamide MEA; coconut fatty acid diethanolamides, and mixtures thereof, being most preferred. Any amount of cationic surfactants or non-ionic surfactants employed in the detergent base are in addition to the amount of the non-ionic surfactant or cationic sufactant, respectively, that may be included in the vesicle bilayer.

Examples of suitable cationic surfactants include, but are not limited to, those set forth in WO 07/26860, as well as the quaternary ammonium surfactants and quaternary amine surfactants that are not only positively charged at the pH of the shampoo composition, which generally is about pH 10 or lower, but also are soluble in the shampoo composition. Preferred cationic surfactants nonexclusively include the n-acylamidopropyl dimethylamine oxides such as cocamidopropylamine oxide sold commercially under the tradename "Incromine Oxide C" available from Croda Inc. Parsippany, New Jersey.

Examples of suitable amphoteric surfactants include, but are not limited to, those set forth in WO 07/26860, i.e., amphocarboxylates, alkyl betaines, amidoalkylbetaines, amidoalkylsultaines, amphophosphates, phosphobetaines, pyrophosphobetains, carboxyalkyl alkyl polyamines, and mixtures thereof. Preferred amphoteric surfactants include amidoalkylbetaines such as cocamidopropyl betaine available commercially from Goldschmidt Chemical Corporation of Hopewell, Virginia under the tradename "Tegobetaine E"; alkyl imidazoline having from about 8 carbon atoms to about 18 carbon atoms in the alkyl group such as Sodium Cocoamphopropionate available commercially from Mona Industries Inc. of Paterson, New Jersey under the tradename "Monateric CA-35".

Examples of suitable soaps include fatty acids reacted with potassium, sodium, ammonium, lithium, triethanol amine bases to form soaps such as sodium cocoate or triethanolamine cocoate.

In a preferred embodiment, the detergent is comprised of a mixture of, based upon the total weight of the detergent, from about 0.1 percent to about 20 percent, and preferably from about 5 percent to about 15 percent anionic surfactants, from about 0 percent to about 10 percent, and preferably from about 1 percent to about 7 percent nonionic surfactants, from about 0 percent to about 5 percent, and preferably from about 0 percent to about 4 percent cationic surfactants, and from about 0.1 percent to about 15 percent, and preferably from about 1 percent to about 10 percent amphoteric surfactants.

In another preferred embodiment, the cleansing composition is comprised of, based upon the total weight of surfactant, from about 50 percent to about 99 percent, and preferably from about 80 percent to about 95 percent, of anionic surfactants preferably selected from the group consisting of alkyl sulfates, alkyl ether sulfates, and mixtures thereof wherein the alkyl group has from about 8 carbon atoms to about 18 carbon atoms, and from about 1 percent to about 20 percent, and preferably from about 5 percent to about 15 percent of amphoteric surfactants, preferably cocamidopropyl betaine.

In another preferred embodiment, the cleansing composition is comprised of, based upon the total weight of surfactant, from about 50 percent to about 99 percent, and preferably from about 70 percent to about 90 percent, of anionic surfactants, preferably those selected from the group consisting of sodium PEG-7 olive oil carboxylate, alkyl sulfates, alkyl ether sulfates, and mixtures thereof wherein the alkyl group has from about 8 carbon atoms to about 18 carbon atoms; from about 1 percent to about 30 percent, and preferably from about 10 percent to about 25 percent of an amphoteric surfactant preferably selected from the group consisting of cocamidopropyl betaine and mixtures thereof; and optionally, from about 0 percent to about 15 percent, and preferably from about 2 percent to about 10 percent of a cationic surfactant such as cocammoniumcarbomoyl chloride.

In a preferred embodiment, the surfactant is used with a suspending agent for purposes of suspending the vesicles or particulate actives such as zinc pyrithione. Examples of suitable suspending agents nonexclusively include: 1) acrylate polymers and copolymers thereof such as the Acrylates/Aminoacrylates C10-30 Alkyl PEG-20 Itaconate copolymer available commercially from National Starch and Chemical Corporation of Bridgewater, New Jersey under the trade name "Structure Plus"; 2) fatty acyl derivatives, wherein the acyl group has the structure VI: 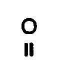 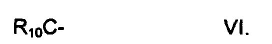 wherein R₁₀ comprises a carbon chain having from about 7 to about 21 carbon atoms that is either saturated or unsaturated and is either substituted or unsubstituted with, for example, hydroxyl groups; 3) esters of long chain fatty acids, wherein the fatty acids have the structure VII: 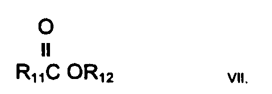 wherein R₁₁ is an alkyl group having from 8 carbon atoms to about 30 carbon atoms, and R₁₂ is an alkyl group having from 8 carbon atoms to about 30 carbon atoms, such as stearyl stearate ; 4) alkyl dimethylamine oxides wherein the alkyl group has from about 8 carbon atoms to about 18 carbon atoms as disclosed in U.S. Patent Re. 34,584, which is incorporated by reference herein in its entirety; 5) methylvinylether/maleic anhydride copolymer crosslinked with 1,9-decadiene PolyVM/MA (PVM/MA decadiene crosspolymer) available from International Specialty Products under the tradename, " Stabileze 06 & QM;" 6) cellulose derivatives such as methylcellulose, hydroxybutyl methylcellulose, hydroxypropylcellulose, hydroxypropyl methylcellulose, hydroxyethyl ethylcellulose, hydroxyethyl cellulose, and mixtures thereof; 7) Distearyl Phthalic Amide available from Stepan Company under the tradename "Stepan SAB-2," and Di(hydrogenated) Tallow Phthalic Amide available from the same under the tradename "Stepan TAB-2"; 8) primary amines having a fatty alkyl group with at least 16 carbon atoms such as palmitate amine and stearamine; 9) polyacrylic acids such as carbomers, which are available from B. F. Goodrich Company under the tradename, "Carbopol"; 10) polysaccharide gums such as xanthan gum; 11) colloidal clays such as benzyl dimethyl hydrogenated tallow ammonium montmorillonite (Bentone 27); 12) colloidal silica; and mixtures thereof. Examples of suitable fatty acyl derivatives include ethylene glycol distearate, ethylene glycol monostearate, and alkanolamides such as cocamide MEA, and mixtures thereof.

Preferred suspending agents include carbomer, hydroxyethyl cellulose, methylvinylether/maleic anhydride copolymer crosslinked with 1,9-decadiene PolyVM/MA (PVM/MA decadiene crosspolymer), and Acrylates/Aminoacrylates C10-30 Alkyl PEG-20 Itaconate Copolymer, with Acrylates/Aminoacrylates C10-30 Alkyl PEG-20 Itaconate Copolymer being most preferred.

The suspending agent is preferably used in an amount effective for suspending the vesicles or particles active agents. Although such amount may vary dependent upon type of benefit agent selected, viscosity of the formulation desired, stability of the formulation, typically the amount of suspending agent may range, based upon the total weight of the detergent, from about 0 percent to about 1 percent, and preferably from about 0.4 percent to about 0.75 percent.

The suspending agent may be simulaneously combined with the detergent, the lipids, the sterols, the benefit agents, and the hydrophilic component. Preferably, the suspending agent may be pre-mixed with the detergent component, and the resulting mixture is combined with the vesicle delivery system components.

We have unexpectedly found that the vesicle delivery system of the present invention is not only effective for delivering the benefit agents into and onto the skin, but it also remains intact and stable in the presence of various detergents.

The vesicle delivery system, when combined with the benefit agent and the detergent, is present in an amount effective to enable a sufficient amount of the benefit agent into and/or onto the skin. While the amount of vesicle delivery system used will vary with the type and amount of benefit agent desired, the intended usage of the final composition. i.e. therapeutic versus maintenance regimen, the amount of detergent present, and the sensitivity of the individual user to the composition, typically the weight ratio of the vesicle bilayer components: benefit agent: detergent may vary from about 1 to about 80 parts, and preferably from about 5 parts to about 30 parts of the vesicle bilayer components, and sterol in the vesicle delivery system : about 0.001 parts to about 20 parts, and preferably from about 0.1 parts to about 5 parts of the benefit agent : about 1 part to about 30 parts, and preferably from about 5 parts to about 20 parts of the detergent.

Another embodiment of the present invention is directed to a composition comprised of the vesicle delivery system and a detergent. We have unexpectedly found that the vesicles of the present invention remain stable and intact in the presence of various detergents, regardless of the presence of the benefit agents.

The vesicle delivery system, when combined with the detergent, is present in an amount effective to remain stable and intact in the presence of the detergent. While the amount of vesicle delivery system used will vary with the type and amount of detergent desired, the intended usage of the final composition, i.e. therapeutic versus maintenance regimen, and the sensitivity of the individual user to the composition, typically the amount of the vesicle bilayer components and detergent may range from, based upon the total weight of the vesicle bilayer components and detergent, about 0.5 percent to about 7 percent, and preferably from about 1 percent to about 5 percent of the vesicle bilayer components, and from about 5 percent to about 25 percent, and preferably from about 8 percent to about 20 percent of the detergent.

The composition of this invention can be formulated in a variety of dosage forms for topical application that include, but are not limited to, for example, washes, baths, lotions, creams, ointments, sprays, aerosols, skin patches, soap, mousses, tonics, gels, solids (e.g. sticks) or the like which is designed to be left on the skin and not washed shortly after application. Alternatively, the composition may be applied to the desired area in the form of, for example, a lotion, cream, gel, soap, shampoo or the like which is designed to be rinsed off within a given amount of time after application.

Another embodiment of the present invention is directed to a method for enhancing the topical application of benefit agents which comprises topically administering to a human or animal a composition, which may optionally contain a detergent, as described above.

While the frequency and amount of the vesicle delivery system to be applied will depend upon, for example, the type and amount of benefit agent available, the intended usage of the final composition, i.e. therapeutic versus maintenance regimen, the amount and type of detergent present, and the sensitivity of the individual user to the composition, typically the composition of the present invention should be topically applied to affected body parts at regular intervals, and preferably from about 2 to about 14 times per week. More preferably, the composition is applied more frequently during the initial stages of treatment, e.g. from about 5 to about 7 times per week until the desired effect is achieved, then less frequently when maintenance is desired, e.g. from about 2 to about 5 times per week.

In a preferred embodiment wherein the composition is incorporated into a shampoo, the shampoo is applied to wet hair, and the hair is washed in accordance with known practices. More preferably, the composition remains on the hair for greater than about 0 to about 10 minutes, and preferably from about 4 to about 7 minutes before rinsing.

An alternative preferred embodiment of the present invention is directed to a method for treating the symptoms and/or the diseases of dandruff, seborrheic dermatitis and/or psoriasis, comprising topically applying the above-described composition, which may optionally include a detergent, to a location desired wherein the benefit agent is comprised of an effective amount of a dandruff treatment agent, a seborrheic dermatitis treatment agent, or a psoriasis treatment agent, respectively. As used herein, "dandruff treatment agent," "seborrheic dermatitis treatment agent," or a "psoriasis treatment agent," respectively, shall include agents capable of treating the symptoms and/or the diseases of dandruff, seborrheic dermatitis, and psoriasis, respectively. By "effective amount," it is meant an amount effective for treating the disease and/or the symptoms associated therewith and preferably may range from, based upon the total weight of the vesicle delivery system and optional detergent, from about 0.001 percent to about 20 percent, and preferably from about 0.01 percent to about 10 percent, and more preferably from about 0.01 percent to about 5 percent.

Examples of benefit agents suitable for treating the symptoms and/or the diseases of dandruff, seborrheic dermatitis and/or psoriasis, respectively, nonexclusively include those set forth above with elubiol, shale oil and derivatives thereof, ketoconazole, coal tar, salicylic acid, zinc pyrithione, selenium sulfide, hydrocortisone, sulfur, menthol, pramoxine hydrochloride, and mixtures thereof being particularly preferred.

An alternative preferred embodiment of the present invention is directed to a method for treating hair loss, such as hair loss resulting from alopecia, comprising topically applying the above-described composition, which may optionally include a detergent, to a desired location wherein the benefit agent is comprised of an effective amount of a hair loss treatment agent such as minoxidil or mixture thereof. As used herein, "hair loss treatment agents" shall include agents capable of growing hair and/or agents capable of preventing the loss of hair. By "effective amount," it is meant an amount effective for treating hair loss and preferably may range from, based upon the total weight of the vesicle delivery system and optional detergent, from about 0.001 percent to about 20 percent, and preferably from about 1 percent to about 5 percent.

Examples of benefit agents suitable for treating hair loss include, but are not limited to potassium channel openers or peripheral vasodilators such as minoxidil, diazoxide, and compounds such as N*-cyano-N-(tert-pentyl)-N'-3-pyridinyl-guanidine ("P-1075") as disclosed in United States Patent No.: 5,244,664, which is incorporated herein by reference; vitamins, such as vitamin E and vitamin C, and derivatives thereof such as vitamin E acetate and vitamin C palmitate; hormones, such as erythropoietin, prostaglandins, such as prostaglandin El and prostaglandin F2-alpha; fatty acids, such as oleic acid; diruretics such as spironolactone; heat shock proteins ("HSP"), such as HSP 27 and HSP 72; calcium channel blockers, such as verapamil HCL, nifedipine, and diltiazemamiloride; immunosuppressant drugs, such as cyclosporin and Fk-506; 5 alpha-reductase inhibitors such as finasteride; growth factors such as, EGF, IGF and FGF; transforming growth factor beta; tumor necrosis factor; non-steroidal anti-inflammatory agents such as benoxaprofen; retinoids such as tretinoin; cytokines, such as IL-6, IL-1 alpha, and IL-1 beta; cell adhesion molecules such as ICAM; glucorcorticoids such as betametasone; botanical extracts such as aloe, clove, ginseng, rehmannia, swertia, sweet orange, zanthoxylum, Serenoa repens (saw palmetto), Hypoxis rooperi, stinging nettle, pumpkin seeds, and rye pollen; other botanical extracts including sandlewood, red beet root, chrysanthemum, rosemary, burdock root and other hair growth promoter activators which are disclosed in DE 4330597 which is incorporated by reference in its entirety herein; homeopathic agents such as Kalium Phosphoricum D2, Azadirachta indica D2, and Joborandi DI; genes for cytokines, growth factors, and male-pattered baldness; antifungals such as ketoconazole and elubiol; antibiotics such as streptomycin; proteins inhibitors such as cycloheximide; acetazolamide; benoxaprofen; cortisone; diltiazem; hexachlorobenzene; hydantoin; nifedipine; penicillamine; phenothaiazines; pinacidil; psoralens, verapamil; zidovudine; alpha-glucosylated rutin having at least one of the following rutins: quercetin, isoquercitrin, hespeddin, naringin, and methylhesperidin, and flavonoids and transglycosidated derivatives thereof which are all disclosed in JP 7002677, which is incorporated by reference in its entirety herein; and mixtures thereof.

Preferred hair loss treatment agents include 6-(l-piperdinyl)-2,4-pyrimidinediamine-3-oxide, N'-cyano-N-(tert-pentyl)-N'-3-pyridinyl-guanidine, finasteride, retinoids and derivatives thereof, ketoconazole, elubiol or mixtures thereof.

Another embodiment of the present invention is directed to a method for removing hair comprising topically applying an effective amount of the above-described composition, which may optionally include a detergent, to a desired area for removing hair, wherein the benefit agent is comprised of an effective amount of a depilatory agent. In a preferred embodiment, the composition contains, based upon the total weight of the composition, from about 0.001 percent to about 20 percent, and preferably from about 0.01 percent to about 5 percent depilatory agent.

Examples of benefit agents suitable for use removing hair nonexclusively include thioglycolate, magnesium thioglycolate, potassium thioglycolate, strontium thioglycolate, and mixtures thereof.

Another embodiment of the present invention is directed to a method for inhibiting hair growth comprising topically applying an effective amount of the above-described composition, which may optionally include a detergent, to a desired area for inhibiting hair growth, wherein the benefit agent is comprised of an effective amount of a hair growth inhibiting agent. In a preferred embodiment, the composition contains, based upon the total weight of the composition, from about 0.001 percent to about 20 percent, and preferably from about 0.01 percent to about 5 percent hair growth inhibiting agent.

Examples of benefit agents suitable for use in inhibiting hair growth include: serine proteases such as trypsin; vitamins such as alpha-tocophenol (vitamin E) and derivatives thereof such as tocophenol acetate and tocophenol palmitate; antineoplastic agents, such as doxorubicin, cyclophosphamide, chlormethine, methotrexate, fluorouracil, vincristine, daunorubicin, bleomycin and hydroxycarbamide; anticoagulants, such as heparin, heparinoids, coumaerins, detran and indandiones; antithyroid drugs, such as iodine, thiouracils and carbimazole; lithium and lithium carbonate; interferons, such as interferon alpha, interferon alpha-2a and interferon alpha-2b; retinoids, such as retinol (vitamin A), isotretinoin: glucocorticoids such as betamethasone, and dexamethosone; antihyperlipidaemic drugs, such as triparanol and clofibrate; thallium; mercury; albendazole; allopurinol; amiodarone; amphetamines; androgens; bromocriptine; butyrophenones; carbamazepne; cholestyramine; cimetidine; clofibrate; danazol; desipramine; dixyrazine; ethambutol; etionamide; fluoxetine; gentamicin, gold salts; hydantoins; ibuprofen; impramine; immunoglobulins; indandiones; indomethacin; intraconazole; levadopa; maprotiline; methysergide; metoprolol; metyrapone; nadolol; nicotinic acid; potassium thiocyanate; propranolol; pyridostimine; salicylates; sulfasalazine; terfenadine; thiamphenicol; thiouracils; trimethadione; troparanol; valproic acid; and mixtures thereof.

Preferred hair growth inhibitory agents include serene proteases, retinol, isotretinoin, betamethoisone, alpha-tocophenol and derivatives thereof, or mixtures thereof.

Another preferred embodiment of the present invention is directed to a method for treating acne and for reducing the signs of aging, i.e. wrinkles, fine lines, and other manifestations of photodamage, comprising topically applying an effective amount of the above-described composition, either with or without detergent, to the skin at a desired area, wherein the benefit agent is comprised of an effective amount of an anti-acne agent or an anti-aging agent, respectively.

Examples of suitable anti-aging agents include, but are not limited to inorganic sunscreens such as titanium dioxide and zinc oxide; organic sunscreens such as octyl-methyl cinnamates and derivatives thereof; retinoids; vitamins such as vitamin E, vitamin A, vitamin C, vitamin B, and derivatives thereof such as vitamin E acetate, vitamin C palmitate, and the like; antioxidants including beta carotene, alpha hydroxy acid such as glycolic acid, citric acid, lactic acid, malic acid, mandelic acid, ascorbic acid, alpha-hydroxybutyric acid, alpha-hydroxyisobutyric acid, alpha-hydroxyisocaproic acid, atrrolactic acid, alpha-hydroxyisovaleric acid, ethyl pyruvate, galacturonic acid, glucopehtonic acid, glucopheptono 1,4-lactone, gluconic acid, gluconolactone, glucuronic acid, glucurronolactone, glycolic acid, isopropyl pyruvate, methyl pyruvate, mucic acid, pyruvia acid, saccharic acid, saccaric acid 1,4-lactone, tartaric acid, and tartronic acid; beta hydroxy acids such as beta-hydroxybutyric acid, beta-phenyl-lactic acid, beta-phenylpyruvic acid; botanical extracts such as green tea, soy, milk thistle, algae, aloe, angelica, bitter orange, coffee, goldthread, grapefruit, hoellen, honeysuckle, Job's tears, lithospermum, mulberry, peony, puerarua, nice, safflower, and mixtures thereof.

Preferred anti-aging agents include retinoids, anti-oxidants, alpha-hydroxy acids and beta-hydroxy acid with retinol and tretinoin being most preferred.

Suitable amounts of anti-aging agents include, based upon the total weight of the vesicle delivery system and optional detergent, from about 0.01 percent to about 10 percent, and preferably from about 0.04 percent to about 5 percent.

Examples of suitable anti-acne agents include, but are not limited to topical retinoids (tretinoin, isotretinoin, motretinide, adapalene, tazarotene, azelaic acid, retinol); salicylic acid; benzoyl peroxide; resorcinol; antibiotics such as tetracycline and isomers thereof, erythromycin, and the anti-inflammatory agents such as ibuprofen, naproxen, hetprofen; botanical extracts such as alnus, arnica, artemisia capillaris, asiasarum root, birth, calendula, chamomile, cnidium, comfrey, fennel, galla rhois, hawthrom, houttuynia, hypericum, jujube, kiwi, licorice, magnolia, olive, peppermint, philodendron, salvia, sasa albo-marginata; imidazoles such as ketoconazole and elubiol, and those described in Gollnick, H et at. 196(I) Dermatology Sebaceous Glands, Acne and Related Disorders, 119-157 (1998), which is incorporated by reference herein, and mixtures thereof.

Preferred anti-acne agents include retinol, elubiol, antibiotics, and salicylic acid, with retinol and tretinoin being most preferred.

Suitable amount of anti-acne agents include, based upon the total weight of the vesicle delivery system and optional detergent, from about 0.01 percent to about 10 percent, and preferably from about 0.04 percent to about 5 percent.

Another preferred embodiment of the present invention is directed to a method for depigmenting the skin comprising topically applying to skin at a desired area the above-described vesicle delivery system containing an effective amount of a depigmentation benefit agent and an optional detergent. Suitable effective amounts of depigmentation agents include, based upon the total weight of the vesicle delivery system and optional detergent, from about 0.01 percent to about 10 percent, and preferably from about 0.04 percent to about 5 percent.

Examples of suitable depigmentation agents include, but are not limited to retinoids such as retinol; Kojic acid and its derivatives such as, for example, kojic dipalmitate; hydroquinone and it derivatives such as arbutin; transexamic acid; vitamins such as niacin, vitamin C and its derivatives; azelaic acid; placertia; licorice; extracts such as chamomile and green tea, and mixtures thereof, with retinol, Kojic acid, and hydroquinone, being preferred.

Further within the scope of the invention are kits that are comprised of the vesicle delivery system, an optional benefit agent, and an optional detergent as well as instructions for their use. In one embodiment, the kit may be comprised of some or all of the materials for forming the vesicle delivery system packaged separately or in pre-mixed combinations as well as instructions explaining the preparation of the delivery system. In yet other embodiments, such kits can further comprise a benefit agent and/or a detergent, wherein the benefit agent is either premixed, i.e. with the vesicle delivery system components or the detergent, or provided in a separate container therefrom.

Preferred sizes of the liposomal vesicles (exclusive of the benefit agent) in the vesicle delivery system may range from about 50 nm to about 5000 nm, with from about 100 nm to about 3000 nm being preferred. One skilled in the art would be capable of modifying the size of the liposome vesicles without undue experimentation such as reduction via sonication. The sonication process in well known in the art and disclosed in, for example, Betageri, G. V., et al., "Preparation of Liposomes," Liposome Drug Delivery Systems, 11 (1993), which is incorporated by reference herein. Alternatively, the size of liposomes could be changed and/or rendered more uniform by extruding the liposomes through a series of straight-bore polycarbonate membranes of varying pore diameters at high pressure, e.g. about 250 psi, as disclosed in, for example, Martin, F. J., "Pharmaceutical Manufacturing of Liposomes," Specialized Drug Delivery Systems: Manufacturing and Production Technology, 267-316 (1990), which is incorporated by reference herein.

When vesicle delivery systems containing the encapsulated benefit agents and mixed with the detergent, were exposed to accelerated stability conditions having a temperature of 4 °C, 30 °C, 40°C and 50°C for a period of about 1 month, and the integrity of the aged vesicle bilayers were evaluated via particle size analysis and freeze-fracture electron microscopy as described in, for example, Betageri, G. V., et al. 1993 " Preparation of Liposomes," in Liposome Drug Delivery Systems, Lanncaster PA: Technomic Publishinh Co., Inc. pp. 33-34, we have unexpectedly found that the characteristics of the detergents have been modified to the extent that they did not destroy the vesicles or its bilayers as they do in other vesicle-containing formulations known in the art. Thus, we surprisingly found that the compositions of the present invention that contained the vesicle delivery systems, the benefit agent, and the detergent not only remained stable upon such accelerated aging conditions but also retained their multi-lamellar structures despite the combination of detergents therewith.

After employing size exclusion chromatography as disclosed in, for example Dowton, S. M., et al, 1993 "Influence of liposomal composition on topical delivery of encapsulated cyclosporin A l. An in vitro study using hairless mouse skin," STP Pharma Sci., 3, 404-407, to determine the amount of entrapment of the benefit agent within the vesicle delivery system both before and after mixing the detergent therewith, we further unexpectedly found that when the benefit agents were inserted into either the hydrophilic component or the bilayer structure of the vesicles, the vesicles were better able to retain the benefit agent upon accelerated aging, even when further combined with the detergent-based cleansers, than those compositions where the benefit agent was external to the vesicle.

We also unexpectedly found that the compositions comprised of benefit agent, detergent, and vesicle delivery system were more effective and efficient than similar vesicle-free compositions in depositing and delivering the benefit agents into and onto the skin, hair and pilosebaceous units. We further surprisingly found that such novel vesicle cleansing formulations substantially target the hair and hair follicles, as opposed to targeting the stratum comeum lipid domain, when delivering the benefit agents to the skin, hair, and pilosebaceous units.

The invention illustratively disdosed herein suitably maybe practiced in the absence of any component, ingredient, or step which is not specifically disdosed herein. Several examples are set forth below to further illustrate the nature of the invention and the manner of carrying it out. However, the invention should not be considered as being limited to the details thereof.

### Examples

### Example 1: Preparation of Vesicle Delivery Systems

Table 1 describes eight vesicle delivery systems used in the following examples. Table 2 provides a short, general description of each vesicle delivery system.

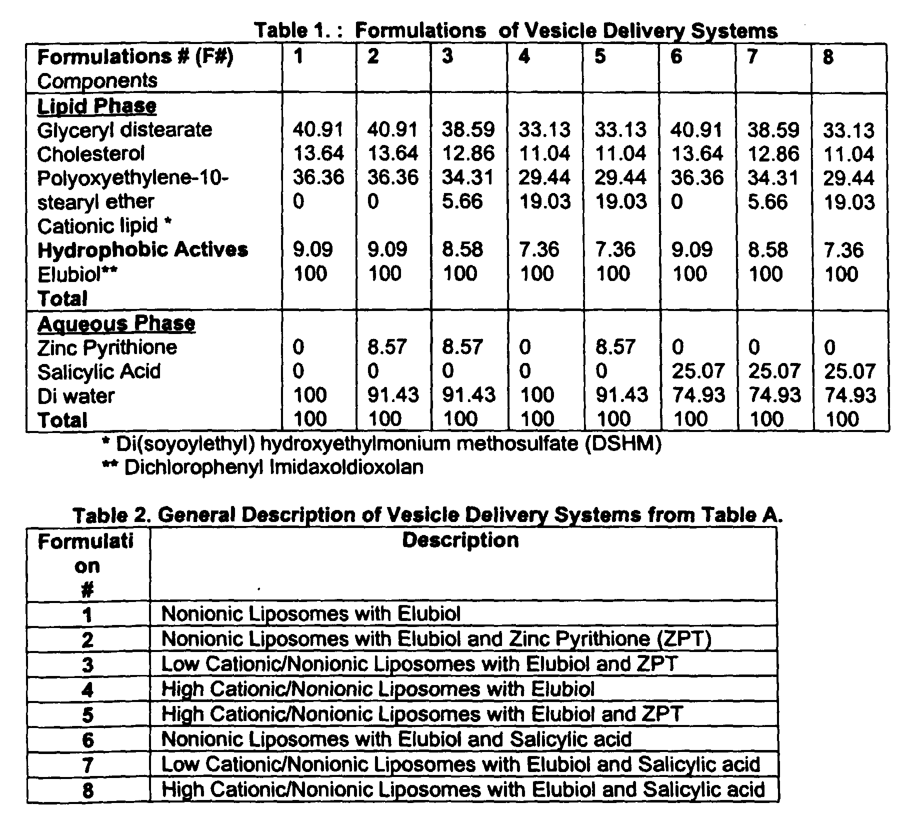

Each of the above systems were made by mixing appropriate amounts of the lipids in a beaker at 75°C until the lipids melted. The resulting melt was then drawn into a syringe, which was preheated in a water-bath to 75°C. A second syringe containing appropriate amounts of the hydrophilic component was preheated in a water-bath to 70°C. The two syringes were then connected via a 3-way metal stopcock. The ratio of aqueous phase to lipid phase was about 70:30 or 7 ml of aqueous phase to 3 ml of lipid phase. After injecting the hydrophilic component into the lipid phase syringe, the resulting mixture was rapidly mixed back and forth between the two syringes several times until the contents cooled to about 25-30°C.

### Example 2: Preparation of Liposomal-Anionic/Amphoteric Shampoo Compositions

Shampoos comprised of the following components as set forth in Table 3 were prepared:

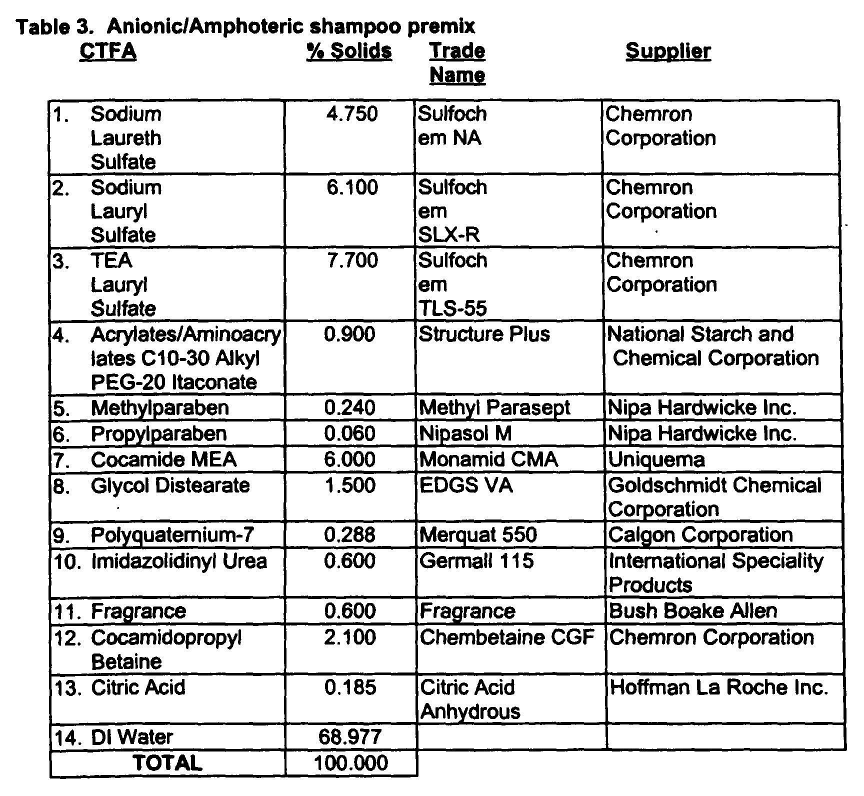

### Preparation of Shampoo Premix:

A vessel was charged with ¾ amount of deionized water (component 14). After combining components 1-4 with mixing for about 5 minutes or until clear, the resulting mixture was heated to 70°C with mixing at 500 rpm for about 20 minutes. Components 5 through 8 were sequentially added thereto with mixing under constant conditions. After cooling the mixture to 45°C, the remaining ingredients 9-12 were sequentially added thereto with mixing at 500 rpm for 20 minutes. After adjusting the mixture to a pH of 6.5 ± 0.5 with citric acid (component 13), the mixture was continuously mixed at 500 rpm until the mixture was about 25-30°C. The remaining amount of deionized water was added to the final volume and was mixed at 500 rpm until uniform.

### Preparation of Vesicle Delivery System Premix s:

The vesicle delivery system of Example 1, F#2 was prepared in accordance with the procedure described in Example 1.

### Preparation of Shampoo Containing Vesicle Delivery System:

Two ml of the vesicle delivery system premix from Example 1, F#2 were added to 10 ml of the shampoo premix with gentle to moderate agitation in a Caframo mixer at 30°C for 20-30 minutes or until uniform.

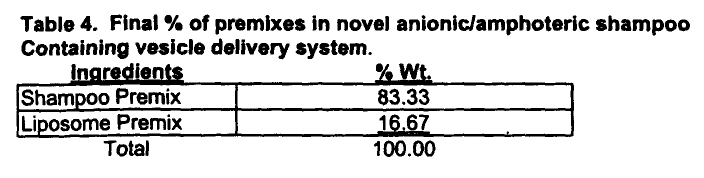

### Example 3: Preparation of Liposomal-Anionic/Amphoteric/Nonionic Shampoo Composition

Shampoos comprised of the following components as set forth in Table 5 were prepared:

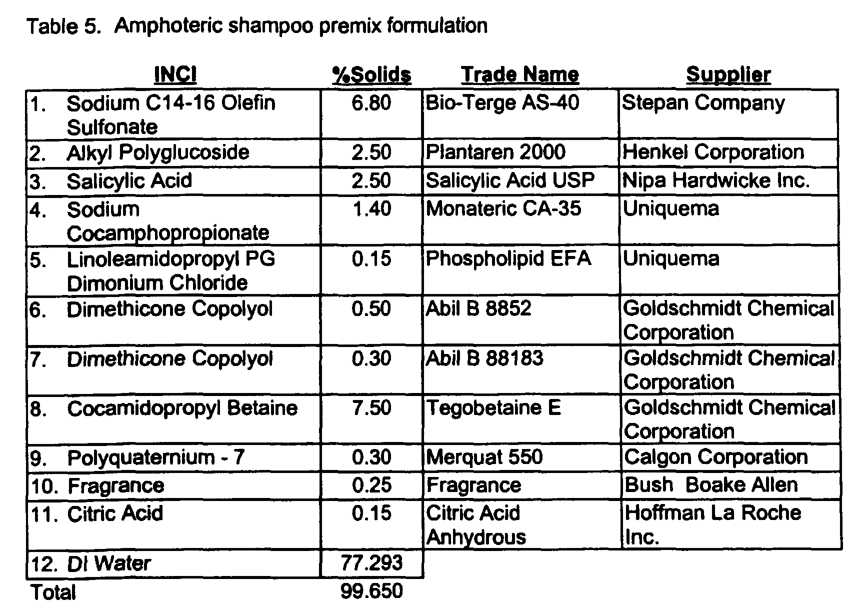

### Preparation of Shampoo Premix:

After charging a vessel with an amount of deionized water (component 12), components 1 and 2 were added thereto sequentially with mixing at about 500 rpm in a Caframo mixer. The mixture was then heated to 70°C. Salicylic acid (component 3) was added thereto with mixing at 500 rpm for 20 minutes under constant temperature. After all the salicylic acid had dissolved, the citric acid was added thereto with mixing under constant conditions. Components 4 through 9 were added sequentially thereto with mixing for 20 minutes at constant conditions. After cooling the mixture to 45°C, the fragrance was added thereto (component 10). An additional amount of citric acid was added thereto to adjust the pH to about 4.0±0.4. The resulting mixture was continuously mixed at 500 rpm until the final batch temperature was about at 25-30°C.

### Preparation of Vesicle Delivery System:

The liposomes of Example 1, F#2 were prepared in accordance with the procedure described in Example 1.

### Preparation of Shampoo Containing Vesicle Delivery System:

Two ml of the liposomal premix from Example 1, F#2 were added to 10 ml of the shampoo premix upon mixing at 500 rpm in a Caframo mixer at 30°C for 20-30 minutes or until uniform.

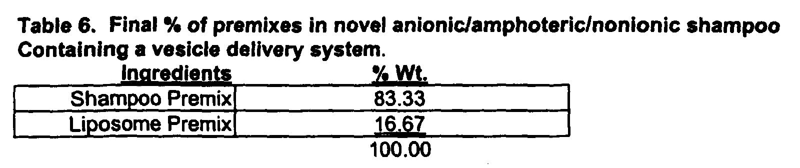

### Example 4: Preparation of Liposomal-Anionic/Amphoteric Shampoo Composition

Shampoos comprised of the following components as set forth in Table 7 were prepared:

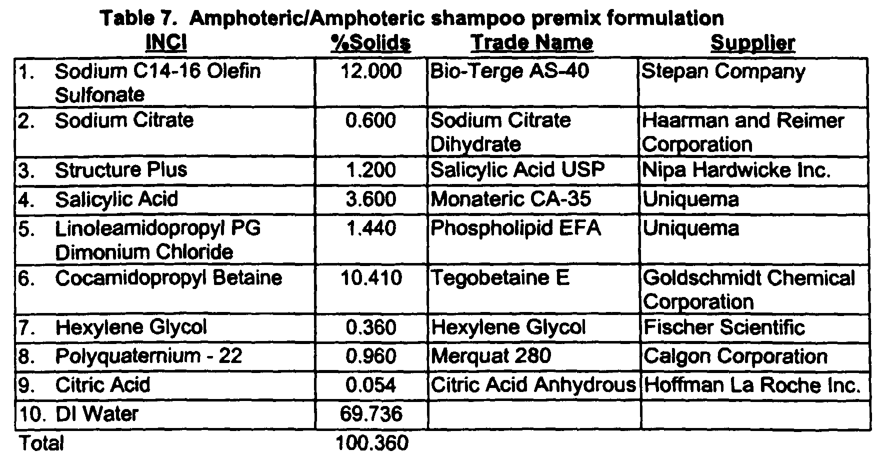

### Preparation of Shampoo Premix:

After charging a vessel with deionized water (component 10), components 2, 3 and 4 were added thereto sequentially with mixing at 500 rpm in a Caframo mixer until uniform. The mixture was then heated to about 70°C with constant agitation. Salicylic acid (component 6) was added thereto under constant conditions. After the salicylic acid dissolved, the mixture was cooled, then components 7 through 10 were added thereto sequentially with mixing at 500 rpm for 20 minutes. After adjusting the pH of the mixture to about 4.0 ± 0.2 with citric acid (component 11), the resulting mixture was mixed under constant conditions then cooled to 25-30°C.

### Preparation of Vesicle Delivery System Premix:

The vesicle delivery system of Example 1, F#2 were prepared in accordance with the procedure described in Example 1.

### Preparation of Shampoo containing Vesicle Delivery System:

Two ml of the vesicle delivery system premix from example 1, F#2 were added to 10 ml of the shampoo premix upon mixing at 500 rpm in a Caframo mixer at 30°C for 20-30 minutes or until uniform.

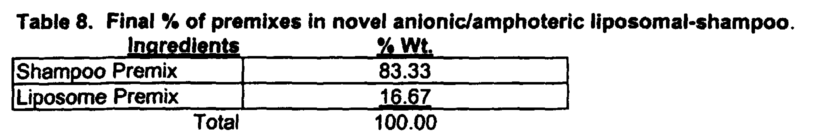

### Example 5: Determination of Skin Permeation

Experiments were conducted to determine the deposition of actives into the skin from various shampoo compositions and liposomal-shampoos compositions. To determine penetration of actives, in vitro skin permeation studies were conducted using non-occluded Franz diffusion chambers.

Human cadaver skin, microtomed to 400 µm, were mounted on Franz diffusion cells containing a receptor medium composed of a citric acid phosphate buffer. The receptor capacity was 5 ml and the cell surface was 0.636 cm². The receptor compartment was maintained at 37°C during the experiment.

In a tube, 50 µl of each formulation as shown in Tables 9 and 11 were diluted with 50 µl of 37°C water. This solution was then rubbed onto the epidermal surface of the mounted skin for 15 seconds, where at it remained for 5 minutes. The solution was then rinsed from the surface three times with 37°C water, and then swabbed twice with dry cotton swabs. At 24 hours after the topical application of the formulation, the surface of the skin was rinsed three times with methanol soaked cotton swabs, and then swabbed three times with three dry cotton swabs. After removing the skin from the diffusion cell, the epidermis and dermis were separated, chopped and placed into separate vials containing a methanol extraction solution and sonicated in a sonicator for 30 minutes. After sonication of the epidermis, dermis and swabs, respectively, each sonicated sample was assayed using a Walters HPLC. Penetration of the active into the skin was calculated based upon a percentage of the applied dose and the amount of active in this case elubiol, delivered into the epidermis or dermis per surface area. The results are shown in Table 9 below:

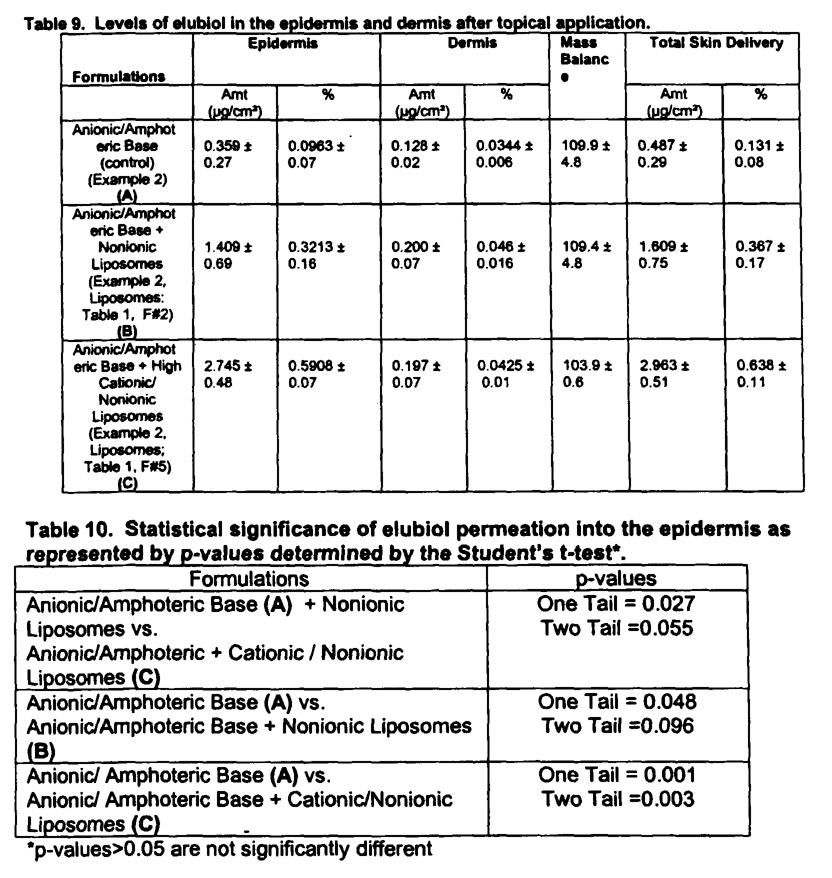

Based upon the results of Tables 9 and 10, this example showed that a control formulation (Formulation A), which contained only the cleansing base, delivered 0.0963% of the applied dose of elubiol into the epidermis. Surprisingly, however, when the formulation containing targeting nonionic vesicle delivery systems was incorporated with the cleansing shampoo base (Formulation B), the percentage of elubiol delivered into the epidermis increased to 0.3213%, a 3.3 fold increase in delivery. When cationic/nonionic vesicle delivery systems were incorporated into the cleansing base (Formulation C), the elubiol permeation into the epidermis surprisingly further increased to 0.5908%, a 6.1 fold increase over the control Formulation A. The levels of elubiol found in the dermis were not significantly different between the three formulations.

This example further demonstrated that the combination of elubiol with cleansing shampoo base and either the nonionic vesicle delivery system or the cationic/nonionic delivery system performed superior with respect to delivering the elubiol to the target site, the epidermis, relative to the combination of elubiol and the base alone. Thus, the composition of this invention affords an effective method of regulating the delivery of hydrophobic actives into the skin, regardless of the presence of detergents.

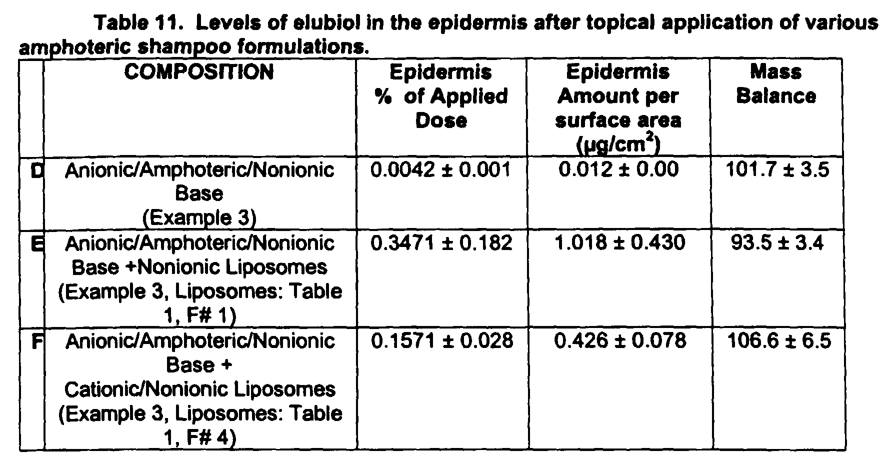

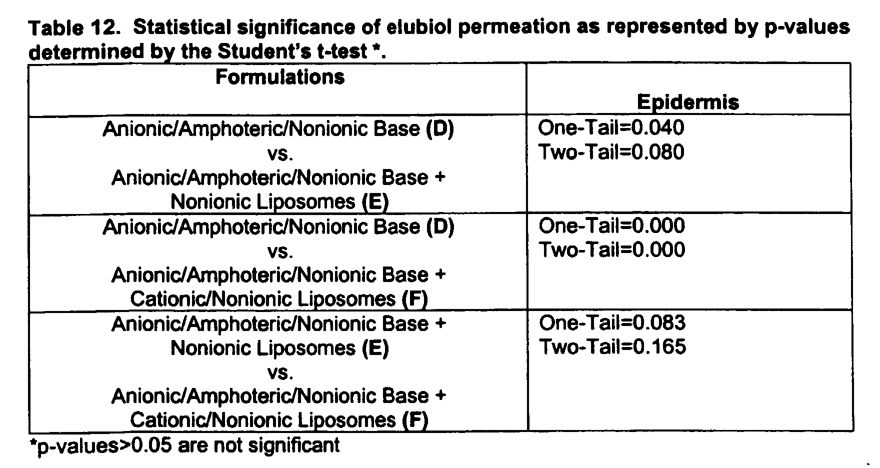

Based upon the data shown in tables 11 and 12 above, this example further showed that a control formulation (Formulation D) containing only the cleansing base delivered only 0.0042% of the applied dose of elubiol into the epidermis. Surprisingly, however, when a formulation containing targeting nonionic vesicle delivery systems was incorporated with the cleansing shampoo base (Formulation E), the percentage of elubiol delivered into the epidermis increased to 0.3471%, almost an 83 fold increase in delivery. When cationic/nonionic vesicle delivery systems were incorporated into the cleansing base (Formulation F), the elubiol permeation into the epidermis surprisingly increased to 0.1571%, a 37 fold increase over the control Formulation D. There were no levels of elubiol found in the dermis of any of the formulation tested above.

As shown in Tables 11 and 12, this example demonstrated that the combination of elubiol, nonionic and cationic/nonionic vesicle delivery systems, and a cleansing shampoo base made up of three different types of surfactants performed superior to the combination of elubiol and the base alone with respect to delivering the elubiol to the target site, the epidermis. Thus, the composition of this invention affords an effective method of regulating the delivery of hydrophobic actives into the skin.

### Example 6: Determination of Entrapment of Agents

The degree of elubiol entrapment in the vesicles was determined using size exclusion chromatography with Sephadex G-75 columns. Details of this procedure is set forth in Dowton, S. M., et al, 1993 "Influence of liposomal composition on topical delivery of encapsulated cyclosporin A l. An in vitro study using hairless mouse skin," STP Pharma Sci., 3, 404-407, which is incorporated by reference herein.

The liposomal formulations from Examples 1, 2 and 3 were tested for elubiol entrapment under accelerated stability conditions. The below formulations were prepared in accordance with previous examples then placed in stability chambers at 50°C for either 2 or 4 weeks. Tables 13 and 14 below shows the level of entrapment of actives for each formulation tested.

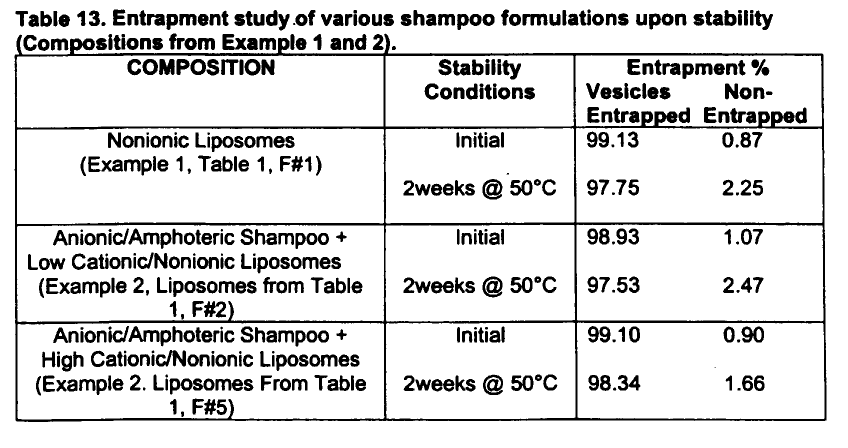

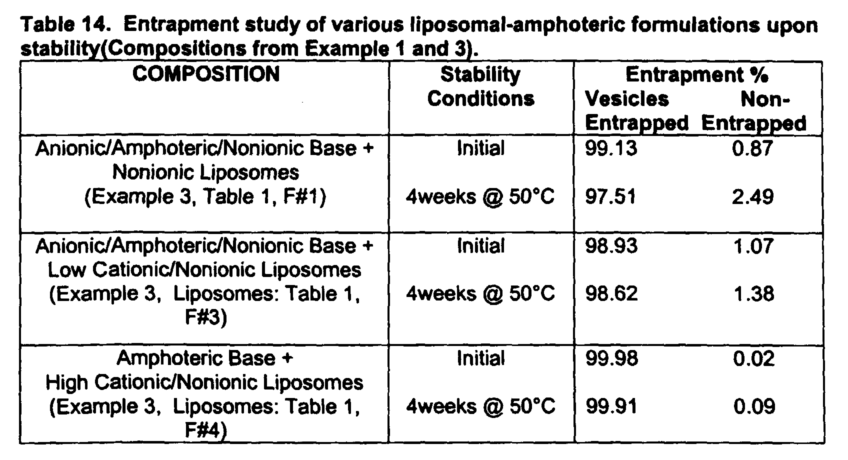

A release of more than 10% of elubiol over 4 weeks under accelerated stability conditions indicated that the vesicles were being destroyed and that elubiol was being released. This example showed that the vesicles containing elubiol were stable regardless of the vesicle composition and cleansing system composition.

### Example 7: Particle Size Analysis

After preparing the compositions in accordance with Examples 1-3, the particle sizes of the resulting formulations were analyzed by inserting 1-ml of a 10-fold dilution of each formulation into a NICOMP 370-submicron particle analyzer using dynamic laser light scattering. The results are presented in Table 15 below, which shows the size ranges based on number-weighted mean diameter of the vesicles in the compositions. The analyzer is unable to accurately detect particle ranges below 30-nm (limit of detection is 20-nm) and vesicles larger than 30 µm (30,000 nm).

The results of the particle size data are further shown in Tables 15 through 21 below.

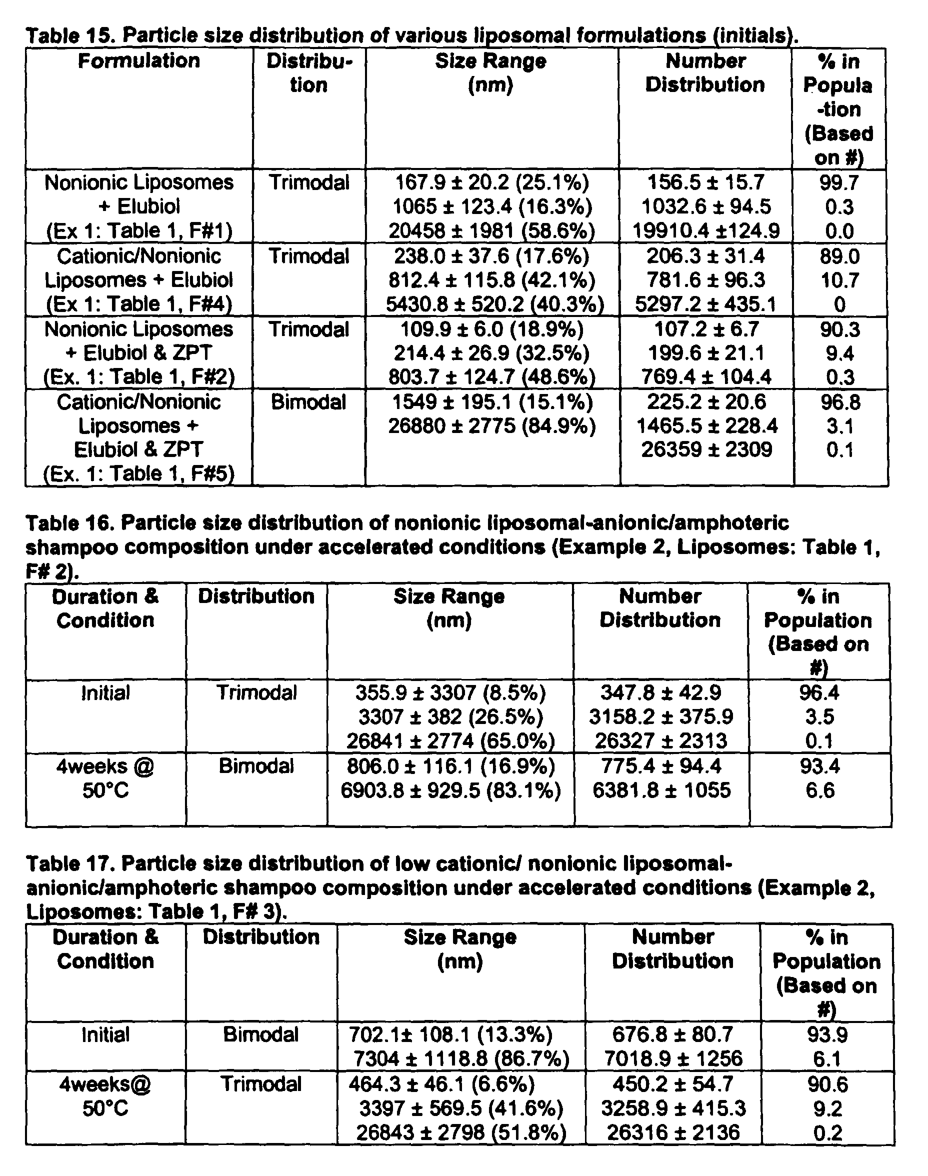

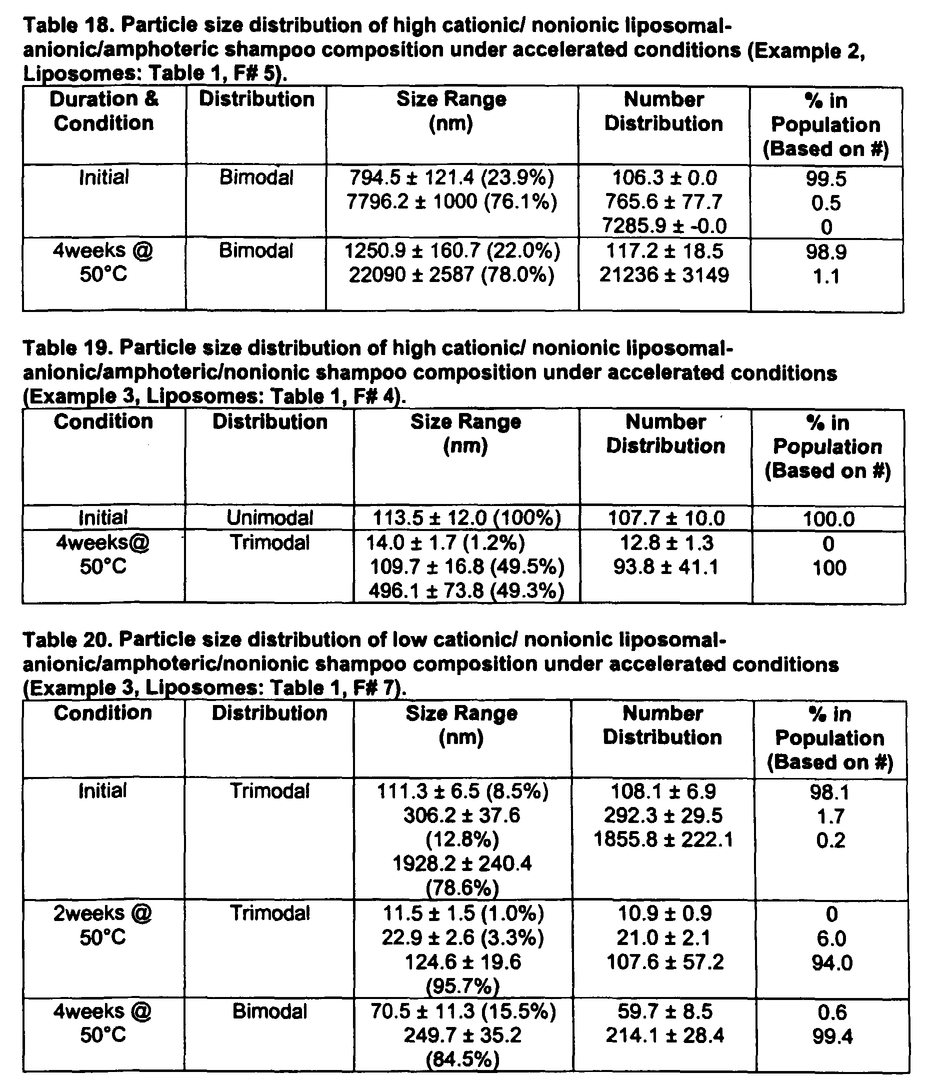

As shown in the tables above, the particle size of the vesicles ranged from 0.1 to 1 µm. The vesicles are considered stable upon accelerated aging with respect to size if over 90% of the vesicles are sized between 0.1 to 1 µm.

The tables above further showed a range of different particle sizes for different liposomes and liposomal-cleansing systems. It is evident that the sizes of the vesicles were relatively constant even though some vesicles doubled their size from 2 weeks at 50°C to 4 weeks at 50°C as shown in Table 20. However, this change may be attributable to the sampling of the vesicles from the container or to the effects caused by the aggregation of two to three vesicles resulting from Van der Waals forces. It was further evident that the vesicles were intact; however, the laser was not capable of distinguishing between two vesicles located very close to one another. Instability and destruction of the vesicles was also evidenced when the particle size increased 3-fold or higher, or if the size changed at least about 10 times in magnitude, i.e. from 100 nm to 1000 nm.

This example illustrated that the particle size of the vesicle delivery system, alone as well as combined with the cleansing base, remained relatively constant over time upon accelerated storage conditions.

### Example 8: Freeze Fracture Microscopy

After the compositions of Examples 1 through 3 were prepared in accordance with previous examples, they were examined using a freeze-fracture transmission electron microscope (FF-TEM). FF-TEM samples of each formulation were prepared in accordance with techniques described in chapter 5 of "Low Temperature Microscopy and Analysis" by Patrick Echlin (1992), which is incorporated by reference herein. The samples were fractured at low temperature and etched at -150°C for purposes of removing a surface layer of water.

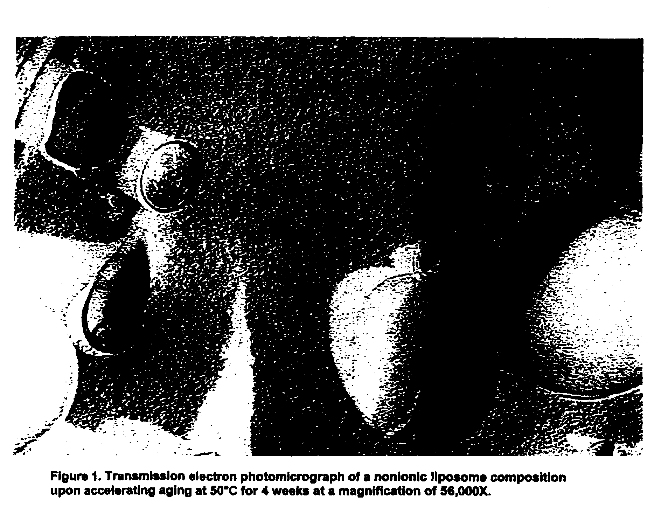

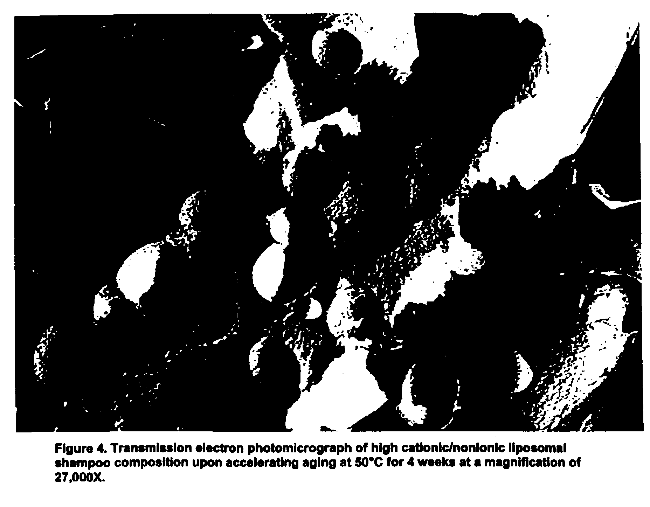

Freeze-fracture photomicrographs of the specimens prepared from the compositions of Examples 1 through 3 are shown in Figure 1, 2, 3 and 4 respectively.

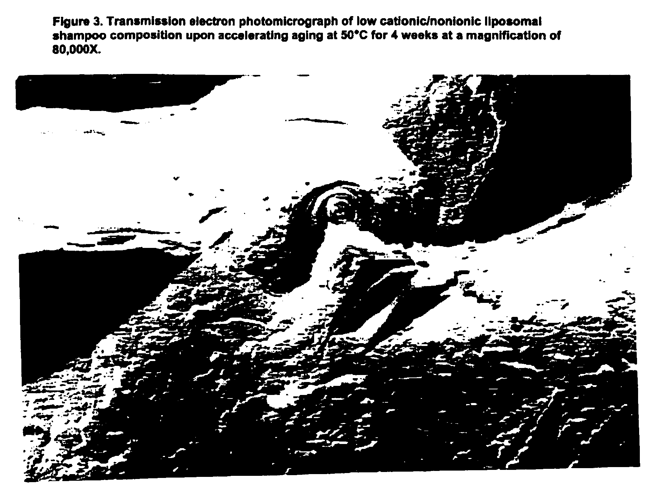

The photomicrograph of Figure 3 was taken of the specimen prepared from the composition of Example 1, which contained only nonionic liposomes. This photomicrograph showed the presence of large bilayered structures ranging in size from 100 nm to 400 nm, which were stable upon product storage at 50°C for 4 weeks.

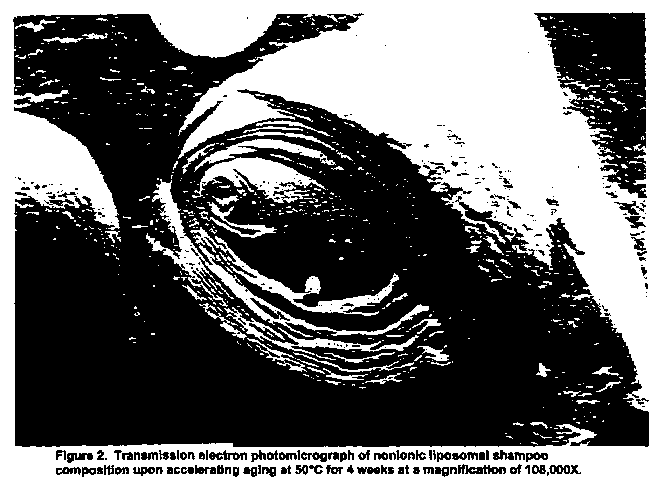

The photomicrograph of Figure 2 was taken of the specimen prepared from the composition of Example 2, which contained nonionic liposomes in a cleansing base. This photomicrograph showed the presence of intact vesicles with many bilayers.

The photomicrograph of Fig. 3 was taken of the specimen prepared from the composition of Example 2, which contained cationic/nonionic liposomes in a cleansing base. It is evident from Fig. 3 that the addition of the cationic component to the vesicle bilayers did not destroy the vesicles.

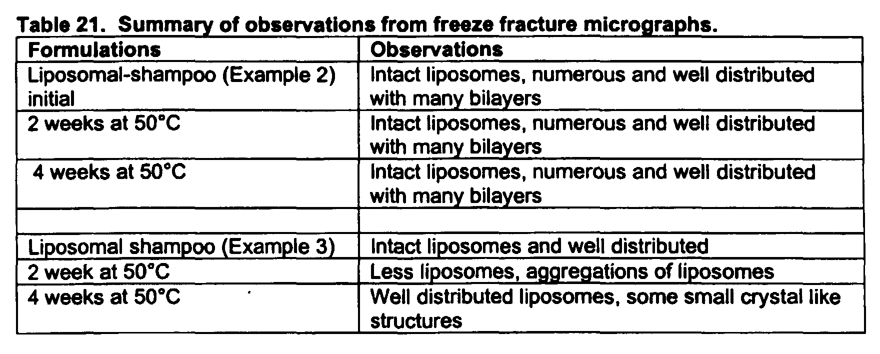

This Example showed that the vesicles remained intact over the accelerated aging storage conditions. This result was very surprising since according to known prior art teaching, it was believed that the interaction of vesicles with detergent cleansing bases would lead to the destruction of the vesicles.

### Example 9: Scanning Electron Microscopy of Hair Tresses

Scanning electron microscopy (SEM) was used to examine hair tresses in order to compare the deposition of solids on individual hair fibers after being shampooed with various cleansing systems containing vesicle delivery systems as set forth in Example 2 (Vesicles table 1, F#5) and "Head & Shoulders" Shampoo (U.S. formula, (L) FW8050), which is available from the Procter & Gamble Company. All of these cleansing systems contained zinc pyrithione in the form of sub-micron to micron sized crystals.

Virgin hair tress sections (∼6-7 g total weight) were washed after application of 0.1 g of each respective shampoo; this procedure was repeated for a total of ten times for each respective shampoo. Each wash cycle consisted of a washing step followed by blow drying until dry after each wash. The hairs were evaluated using a Zeiss SEM on 1-inch long sections taken from the middle and ends of each tress.

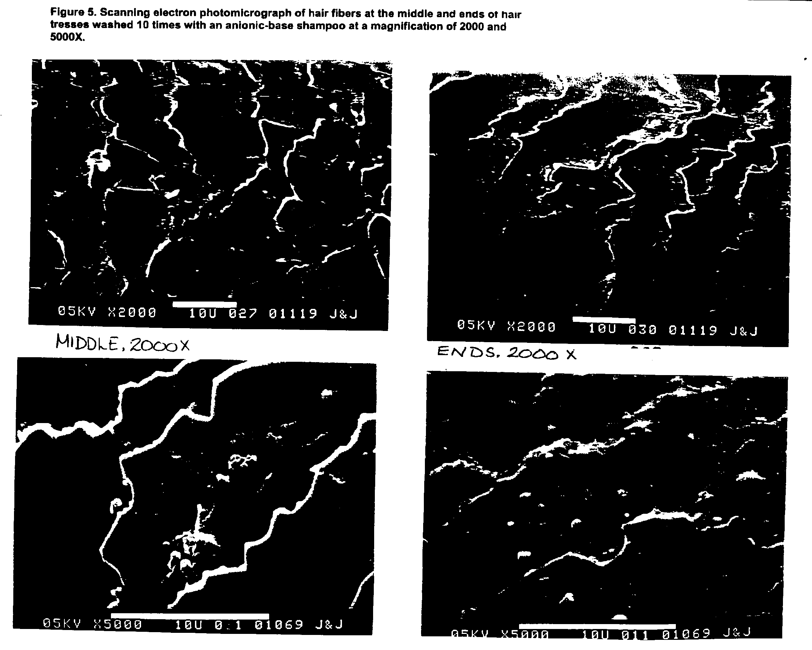

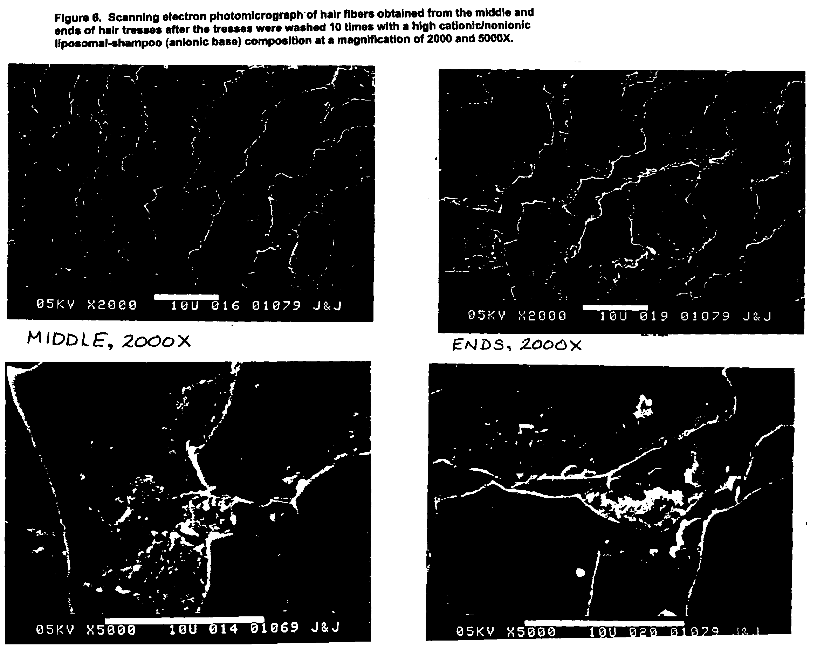

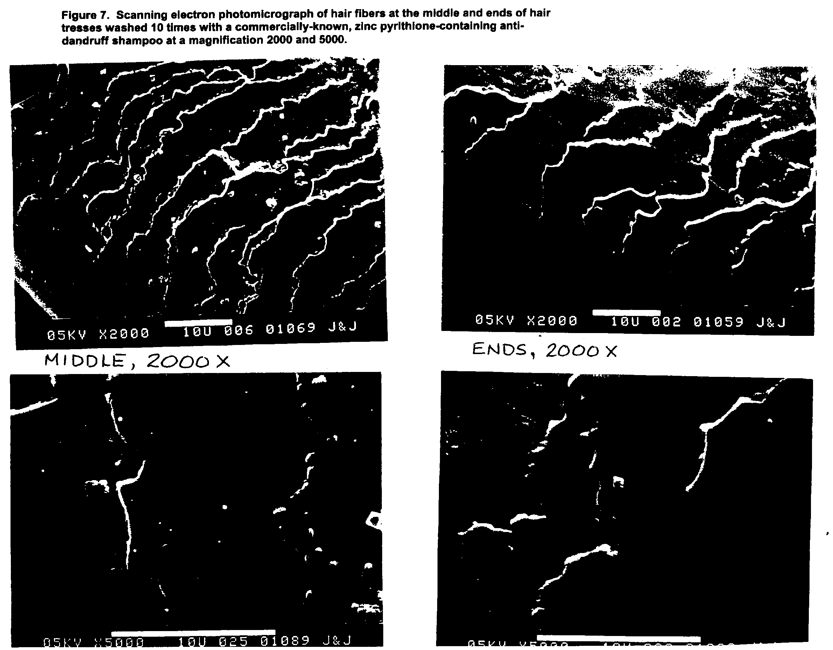

Figure 5 through 7 illustrate the SEM micrographs of the washed hair tresses. From these SEM micrographs it was evident that the solid-depositing ability of the shampoos, in downward order, is as follows: Liposomal- Shampoo (Ex. 2, Liposomes: Ex. 1. Table 1, F#5)(Figure 6)>> Shampoo base alone (Example 2)(Figure 5)> Head & Shoulders (Figure 7).

As shown in Figure 7, the majority of the solids deposited on the hair tresses treated with the Head & Shoulders formulation consisted of submicron to 2-micron size particles and appeared to be uniformly distributed on the cuticles. While not wishing to be bound by this theory, it is believed that solids found on tresses shampooed with the shampoo base alone (Example 2) and vesicle delivery system-shampoo (Example 2, Vesicles: Ex. 1. Table 1, F#5) consisted of particulates in a matrix of elubiol and Polyquaternium-7 and, in the case of the vesicle delivery system-shampoo, additional vesicle components. Therefore, the latter formula appeared to be more uniformly bound to the cuticle relative to the Head and Shoulders formula. This is relevant because it is believed that components bound to the cuticle are more beneficial in the treatment of diseases associated with the hair and scalp due to the superior deposition of benefit agents thereto.

### Example 10: Assay of Hair Tresses Treated

The hair tresses treated above in Example 9 for the scanning electron microscopy study were extracted with methanol, then the amount of elubiol retained on and in the hair fibers was established. Each set of hair tresses was cut into 1.5-cm long segments in the middle and at the ends of the respective sets of hair tresses. After the hairs from the cut tresses were weighted and extracted with methanol, they were then assayed using the HPLC technique set forth in Example 4. The deposition of the active on and in the hair fibers was calculated based upon an amount of active per weight of hair and the amount of active deposited per total surface of the hairs there were assayed. The diameter of the hair was assumed to be 50 µm.

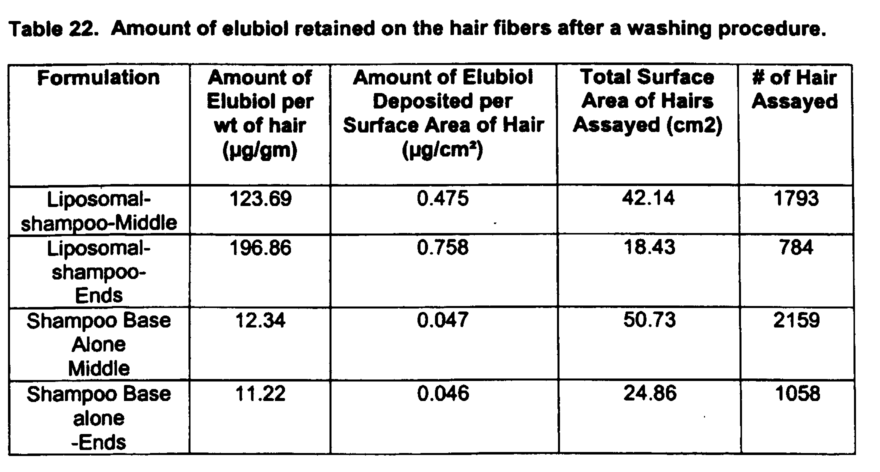

From the results set forth in Table 22, it is evident that the nonionic-cationic cleansing system containing the vesicle delivery system of Example 2 deposited from 10 to 16 times more elubiol into and onto the hair fibers in comparison to the amount deposited by the cleansing system alone of Example 2. This Example also showed that after the hair was treated with the liposomal-shampoo (Example 2, Liposomes: Ex. 1. Table 1, F#5), a greater amount of elubiol was deposited at the ends versus the middle of the hair tresses.

This Example showed that the addition of vesicle delivery systems containing entrapped elubiol into cleansing base compositions resulted in significantly improved deposition of elubiol onto the hair fibers, relative to the deposition achieved by compositions containing only the elubiol and cleansing base alone.

### Example 11: Hamster Ear Model Pilosebaceous Deposition Studies

The hamster ear pilosebaceous units have been shown to have anatomical and physiological similarities to human sebaceous glands by Plewig and Luderschmidt, J. Invest. Dermatol. 68: 171-176, (1977) and Matias and Orentreich, J. Invest. Dermatol. 81: 43-46, (1983), and thus, provide an appropriate model for examining the extent and rate of deposition of clinically potent sebum reducing active, such as elubiol, into the pilosebaceous structures.

Male golden Syrian hamsters (12 weeks old) were obtained from Charles River Breeding Laboratories and housed in cages under a 14 hour light and a 10 hours dark cycle for two weeks in order to maximize their androgen-dependent sebaceous glands activity and thus control their gland size. Four hamsters were used for each formulation tested. The dosing protocol consisted of treating each ear with 10 µl of a formulation as set forth in Table 23 below, rubbing in the formulation for 30 seconds, then rinsing the formulation from the ear with 5 ml of 37°C water; this procedure repeated for a total of two times. This dosing treatment was performed once a day for three days, and the experiment was ended 24 hours after the last dose. Each animal was anesthetized for a total of 1.5 hours during the dosing period with ketamine/xylazine (IP injection).

Table 23 below shows the formulations tested in the study.

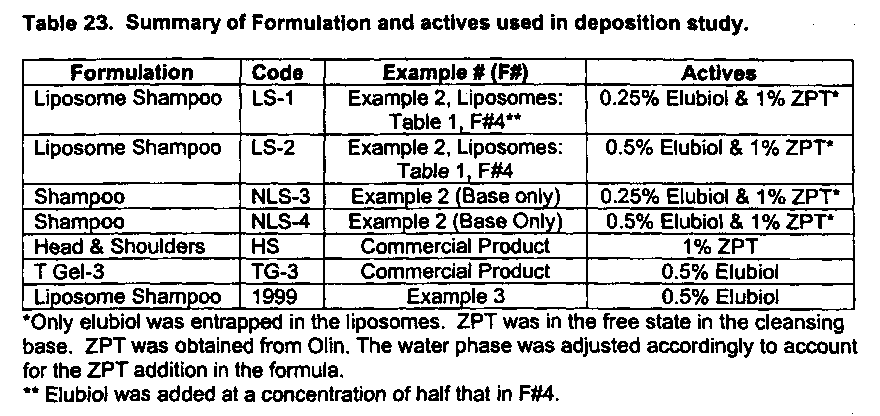

The ears of each hamster were excised at their base, and then the excess hair was trimmed with surgical scissors. The ventral surface of the ears was then swabbed several times with dry swabs. The surface of the skin was then tape stripped 30-50 times with Scotch tape (#810, 3M Company, St. Paul, MN) for purposes of to removing the stratum comeum. A shiny and glossy appearance indicated that removal of the stratum corneum was complete. After complete removal of the stratum comeum, each stripped ear was dissected such that the ventral ear and the dorsal ear were separated. The dissection procedure was carried out carefully so as to assure that the cartilage between the ventral and the dorsal portions of the ear was left in contact with the dorsal ear. The ventral ear section was then placed on a glass slide and the pilosebaceous units were removed with a scalpel. The removal of sebaceous glands was then validated by examining the ear under a Nikon Daphot inverted light microscopy before and after the procedure. The absence of the pilosebaceous units was confirmed by the presence of holes in the ventral tissue. The pilosebaceous units were collected and assayed for elubiol and zinc pyrithione content.

It is evident from the table below that the vesicle delivery system-containing shampoos (Formulations A and B) delivered significantly more elubiol into the pilosebaceous units than that delivered by the shampoo bases alone (Formulations C and D). The vesicle delivery system-containing-cleansing base of Example 3 (System 1999(E)) also delivered 98 times more elubiol into the glands than that achieved by the T-Gel 3 base alone.

This Example further showed that the most effective composition for delivering elubiol into the pilosebaceous units was the vesicle delivery system-containing the anionic/ amphoteric/nonionic base of Example 3, followed by the vesicle delivery system-containing anionic/amphoteric base of Example 2, with a delivered elubiol content of 0.5 % and 0.25%, respectively.

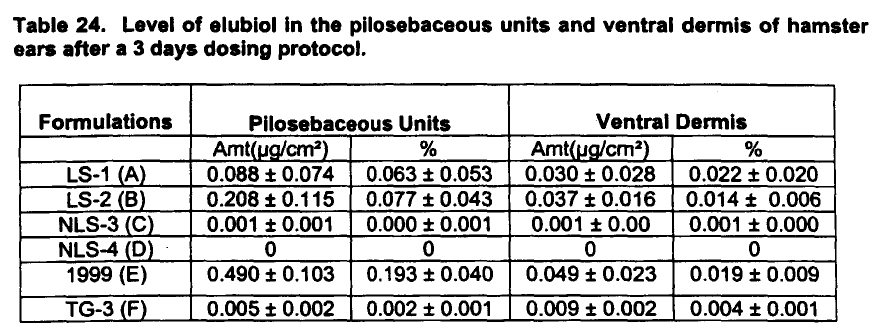

This Example further showed that a similar trend was observed for the delivery of zinc pyrithione ("ZPT") into the pilosebaceous units. Even though ZPT was not entrapped in the vesicles, it was surprisingly apparent the incorporation of the vesicles alone with free ZPT in the external phase enhanced the delivery of ZPT into the glands.

This Example further showed that all of the vesicle delivery system-shampoos performed better than those shampoos lacking vesicle delivery systems with respect to ZPT delivery into the pilosebaceous units. The vesicle delivery system-containing shampoos of Example 2 (Liposomes:Table 1, F4**) and Example 2 (Liposomes:Table 1, F4)(see Table 23) delivered 1.6 and 3.8 times more ZPT, respectively, into the pilosebaceous units in comparison to the amount delivered by the Head and Shoulders base.

This Example also showed that the Head and Shoulders base delivered ZPT into the ventral dermis, which is typically not a desirable effect since delivery into the dermis may lead to irritation problems over an extended period of time.

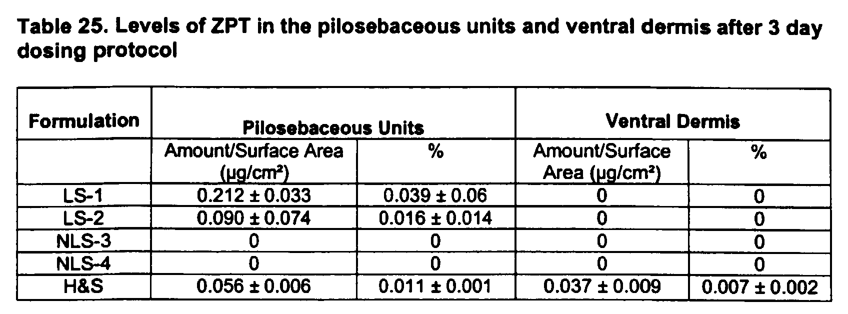

This Example showed that when vesicles entrapped with elubiol are added to a cleansing base, the amount of elubiol delivered into the pilosebaceous units (sebaceous glands and canal) was tremendously increased relative to the amount of elubiol delivered by the elubiol-containing cleansing base alone. In addition, when ZPT was added to such formulations containing vesicles with entrapped elubiol, the delivery of ZPT into the pilosebaceous glands was increased relative to the amount of ZPT delivered by a vesicle-free formulation containing ZPT with elubiol.

## Claims

1. A composition for enhancing the topical application of benefit agents comprising, based upon the total weight of the composition:
A. a vesicle delivery system comprised of:
i. a vesicle bilayer comprised of:
a. at least one first dual chain lipid;
b. optionally, at least one first single chain lipid; and
c. at least one sterol; and
ii a hydrophilic carrier contained within the vesicle bilayer; and
B. an effective amount of the benefit agent.

2. The composition of claim 1 wherein the vesicle delivery system further comprises at least one second dual chain lipid and/or at least one second single chain lipid.

3. The composition of claim 1 or claim 2 wherein the first dual chain lipid is a cationic dual chain lipid, a nonionic dual chain lipid or a mixture thereof.

4. The composition of claim 3 wherein the first dual chain lipid is a nonionic dual chain lipid which is a glyceryl diester, an alkoxylated amide or a mixture thereof.

5. The composition of any one of claims 1 to 4 wherein the first single chain lipid is a non-ionic single chain lipid, a cationic single chain lipid or a nonionic/cationic single chain lipid.

6. The composition of claim 5 wherein the first single chain lipid is a glyceryl monoester; a polyoxyethylene fatty ether wherein the polyoxyethylene head group has from 2 to 100 groups and the fatty acid tail group has from 10 to 26 carbons atoms; an alkoxylated alcohol wherein the alkoxy group has from 1 to 200 carbon atoms and the fatty alkyl group has from 8 to 30 carbon atoms; an alkoxylated alkyl phenol wherein the alkoxy group has from 1 to 200 carbon atoms and the fatty alkyl group has from 8 to 30 carbon atoms; a polyoxyethylene derivative of a polyol ester; an alkoxylated acid wherein the alkoxy group has from 1 to 200 carbon atoms and the fatty alkyl group has from 8 to 30 carbon atoms; or a mixture thereof.

7. The composition of any one of claims 1 to 6 wherein the sterol is phytocholesterol, hydrocortesone, alpha-tocopherol, betasitosterol, bisabolol or a mixture thereof.

8. The composition of any one of claims 1 to 7 wherein the benefit agent is elubiol, 6-(1-piperidinyl)-2,4-pyrimidinediamine-3-oxide, finasteride, ketoconazole, salicylic acid, zinc pyrithione, coal tar, benzoyl peroxide, shale oil or a derivative thereof, selenium sulfide, hydrocortisone, sulfur, menthol, pramoxine hydrochloride tricetylammonium chloride polyquaternium 10, panthenol, panthenol triacetate, vitamin A or a derivative thereof, vitamin B or a derivative thereof, vitamin C or a derivative thereof, vitamin D or a derivative thereof, vitamin E or a derivative thereof, vitamin K or a derivative thereof, keratin, lysine, arginine, hydrolyzed wheat protein, hydrolyzed silk protein, octyl methoxycinnamate, oxybenzone, minoxidil, titanium dioxide, zinc dioxide, retinol, erythromycin, tretinoin or a mixture thereof.

9. The composition of any one of claims 1 to 8 in the form of a lotion, cream, ointment, mousse, bath, spray, aerosol, skin patch, gel, soap, tonic, toner or shampoo.

10. The composition of any one of claims 1 to 9 which additionally comprises:
C. a detergent.

11. The composition of any one of claims 1 to 10 further comprising a suspending agent.

12. The composition of any one of claims 1 to 11 wherein the benefit agent is a hair loss treatment agent.

13. The composition of claim 12 wherein the hair loss treatment agent is minoxdil, N''-cyano-N- (tert-pentyl)-N'-3-pyridinyl-guanidine, diazoxide, vitamin E, vitamin C, vitamin E acetate, vitamin C palmitate; erythropoietin; prostaglandin E1, prostaglandin F2-alpha; oleic acid; heat shock protein 27 ("HSP 27") heat shock protein 72 ("HSP 72"); verapamil HCL, nifedipine, diltiazemamiloride, cyclosporin, Fk-506; finasteride, 17-beta estradiol, EGF, FGF, benoxaprofen, tretinoin, Il-6, Il-1alpha, IL-1beta ICAM, betametasone, aloe, clover, ginseng, rehmannia, swertia, sweet orange, zanthoxylum, elubiol, ketoconazole, zinc pyrithione; streptomycin; cycloheximide; or a mixture thereof.

14. The composition of any one of claims 1 to 11, wherein the benefit agent is a hair growth inhibiting agent.

15. The composition of any one of claims 1 to 11, wherein the benefit agent is an anti-aging active agent.

16. The composition of any one of claims 1 to 11, wherein the benefit agent is an anti-acne active agent.

17. The composition of any one of claims 1 to 11 wherein the benefit agent is a depigmentation active agent.

18. The composition of any one of claims 1 to 11, wherein the benefit agent is an anti-dandruff agent, an anti-seborrheic dermatitis agent, an anti-psoriasis agent or a mixture thereof.

19. The composition of any one of claims 1 to 11, wherein the benefit agent is a depilatory agent.

20. The composition of any one of claims 1 to 18 for use in enhancing the topical application of a benefit agent.
